# EUROPÄISCHE PATENTSCHRIFT

(11) **EP 2 688 603 B1**
(45) Veröffentlichungstag und Bekanntmachung des Hinweises auf die Patenterteilung: **07.06.2023**
(21) Anmeldenummer: 12711787.7
(22) Anmeldetag: 21.03.2012
(51) Int. Cl.: A61J 1/06, A61M 5/31, A61M 5/34, A61M 5/00, A61J 1/16, A61M 5/28

(54) **BEHÄLTER, HALTEVORRICHTUNG, HALTESYSTEM UND INJEKTIONSHILFE**
CONTAINER, HOLDING DEVICE, HOLDING SYSTEM AND INJECTION AID
RÉCIPIENT, DISPOSITIF DE RETENUE, SYSTÈME DE RETENUE ET AUXILIAIRE D'INJECTION

(30) Priorität: 21.03.2011 DE 102011015112
(43) Veröffentlichungstag der Anmeldung: 29.01.2014
(73) Patentinhaber: Vetter Pharma-Fertigung GmbH & Co. KG, 88212 Ravensburg (DE)
(72) Erfinder: GLOCKER, Joachim, 88214 Ravensburg (DE); ROEDLE, Tilman, 88364 Wolfegg (DE)
(74) Vertreter: Kordel, Mattias
(86) Internationale Anmeldenummer: PCT/EP2012/001304
(87) Internationale Veröffentlichungsnummer: WO 2012/126636

(56) Entgegenhaltungen:
- EP-A1- 2 119 463
- WO-A1-03/094999
- WO-A1-2011/015896
- US-A- 2 833 007
- US-B1- 6 569 127

## Beschreibung

Die Erfindung betrifft eine Haltevorrichtung für Behälter gemäß Oberbegriff des Anspruchs 1.

Behälter für pharmazeutische Zubereitungen, Haltevorrichtungen und Haltesysteme für solche Behälter sowie Injektionshilfen sind bekannt. Die Haltevorrichtungen können beispielsweise als Trägersystem für ein Haltesystem für pharmazeutische Behälter ausgebildet sein oder von einer Injektionshilfe umfasst sein. Die Behälter weisen einen Grundkörper auf und sind bevorzugt als Spritze, Karpule, Phiole (Vial) oder bevorzugt auch als Doppelkammersystem ausgebildet. Typischerweise werden solche Behälter der pharmazeutischen Industrie zum Befüllen mit pharmazeutischen Zubereitungen wie beispielsweise Medikamenten bereits gewaschen und/oder sterilisiert angeliefert. Dazu sind vorzugsweise mehrere Behälter in einem Haltesystem zusammengefasst. Sie sind mehr oder weniger lose in einem Trägersystem angeordnet, so dass beim Transport Relativbewegungen zwischen den Behältern und dem Trägersystem einerseits und den einzelnen Behältern andererseits möglich sind. Dies kann zu kosmetischen Defekten an den Behältern führen. Sind diese als Doppelkammersysteme ausgebildet, müssen sie gewendet werden, um beide Kammern füllen zu können. Aufgrund der losen Anordnung in dem Trägersystem müssen die Behälter hierzu aus demselben entnommen und in eigens hierfür vorgesehene Magazine einsortiert werden. Dies bedeutet einen zusätzlichen Arbeitsschritt, außerdem ist es möglich, dass bei einem Greifen der Behälter zum Umsortieren kosmetische Defekte an denselben verursacht werden. Behälter, die als Karpulen ausgebildet sind, werden häufig in Zusammenhang mit Injektionshilfen, beispielsweise sogenannte Autoinjektoren oder Pens eingesetzt. Die Injektionshilfe umfasst ein Gehäusevorderteil zur Aufnahme der Karpule und ein Mechanikteil, welches eine Aktivierungs- und Auslösemechanik umfasst. Eine Karpule kann nicht ohne das Gehäusevorderteil mit dem Mechanikteil verbunden werden. Insgesamt ist es demnach bei bekannten Behältern von Nachteil, dass diese nicht in geeigneter Weise beispielsweise mit einem Trägersystem oder mit einem Mechanikteil eine Injektionshilfe, allgemein einer Haltevorrichtung verbunden werden können, so dass sie sicher und fest an dieser gehalten werden.

Es ist daher Aufgabe der Erfindung, eine Haltevorrichtung zu schaffen, die die genannten Nachteile nicht aufweist.

Diese Aufgabe wird gelöst, indem eine Haltevorrichtung für Behälter für pharmazeutische Zubereitungen mit den Merkmalen des Anspruchs 1 geschaffen wird.

Dieser zeichnet sich durch mindestens ein Haltemittel aus, welches so ausgebildet ist, dass es mit einem korrespondierenden Haltemittel einer Haltevorrichtung für den Behälter zusammenwirken kann, um den Grundkörper sicher an der Haltevorrichtung zu halten. Das Haltemittel ist in Bezug auf den Grundkörper so vorgesehen, dass in den Grundkörper Haltekräfte eingeleitet werden können, so dass letztlich der Behälter sicher an der Haltevorrichtung gehalten wird. Dadurch ist es möglich, diesen derart beispielsweise mit einem Trägersystem eines Haltesystems zu verbinden, dass keine unerwünschten Relativbewegungen zwischen dem Behälter und dem Trägersystem einerseits und verschiedenen in dem Trägersystem angeordneten Behältern andererseits auftreten können. Außerdem ist der Behälter zusammen mit dem Trägersystem handhabbar. Sind mehrere Behälter an dem Trägersystem angeordnet, muss lediglich das Trägersystem gegriffen, transportiert und gewendet werden - letzteres insbesondere in Zusammenhang mit der Befüllung von Doppelkammersystemen - ohne dass einzelne Behälter aus dem Trägersystem aussortiert werden müssen. Aufgrund des Haltemittels kann ein solcher, beispielsweise als Karpule ausgebildeter Behälter auch mit dem Mechanikteil einer Injektionshilfe verbunden werden, ohne dass ein Gehäusevorderteil zur Aufnahme des Behälters nötig ist.

Bevorzugt wird ein Behälter, bei dem das Haltemittel mindestens einen Vorsprung und/oder mindestens eine Ausnehmung umfasst. Der Vorsprung und/oder die Ausnehmung ist/sind vorzugsweise an einer Umfangsfläche des Grundkörpers vorgesehen. Der Vorsprung und/oder die Ausnehmung kann/können mit mindestens einem korrespondierenden Vorsprung und/oder mindestens einer korrespondierenden Ausnehmung so zusammenwirken, dass vorzugsweise eine formschlüssige Verbindung des Behälters mit der Haltevorrichtung entsteht.

Bevorzugt wird beispielsweise ein Behälter, bei dem das Haltemittel so ausgebildet ist, dass es mit einem korrespondierenden Haltemittel nach Art eines Bajonettverschlusses zusammenwirken kann. Das Haltemittel kann also beispielsweise im Wesentlichen aufeinander senkrecht stehende Nuten umfassen, in die entsprechend ausgebildete Vorsprünge des korrespondierenden Haltemittels eingreifen, um nach Art einer Steck-Dreh-Mechanik eine Verbindung zu bewirken, wie sie ein Bajonettverschluss typischerweise bereitstellt. Selbstverständlich ist es ebenso möglich, dass das Haltemittel des Behälters die entsprechenden Vorsprünge aufweist, welche in im Wesentlichen senkrecht aufeinander stehende Nuten des korrespondierenden Haltemittels eingreift.

Bevorzugt wird auch ein Behälter, bei dem das Haltemittel an einem proximalen Ende des Grundkörpers vorgesehen ist. Ist der Behälter als Spritze, Karpule oder Doppelkammersystem ausgebildet, weist er ein proximales und ein distales Ende auf. Das proximale Ende ist dabei besonders geeignet, um ein Haltemittel vorzusehen. Besonders bevorzugt wird das Haltemittel in einem Abstand zum proximalen Ende des Grundkörpers vorgesehen.

Es wird auch ein Behälter bevorzugt, der ein Verbindungselement aufweist. Dieses ist so ausgebildet, dass es ein Werkzeug formschlüssig aufnehmen kann. Das Werkzeug dient dazu, eine Relativdrehung zwischen einer Haltevorrichtung und dem Behälter zu bewirken, um diesen an der Haltevorrichtung zu ver- oder entriegeln. Dazu ist das Verbindungselement so ausgebildet, dass eine Drehmomentübertragung von dem Werkzeug auf den Grundkörper des Behälters möglich ist, so dass eine Verbindung zwischen dem Haltemittel und einem korrespondierenden Haltemittel bewirkt werden kann.

Schließlich wird ein Behälter bevorzugt, der als Spritze, Karpule, Phiole (Vial) oder bevorzugt als Doppelkammersystem ausgebildet ist.

Weitere bevorzugte Ausführungsbeispiele ergeben sich aus den Unteransprüchen.

Die Aufgabe wird auch gelöst, indem eine Haltevorrichtung mit den Merkmalen des Anspruchs 1 geschaffen wird. Diese weist mindestens eine Aufnahme für einen Behälter auf und zeichnet sich dadurch aus, dass die Aufnahme mindestens ein Haltemittel umfasst, welches so ausgebildet ist, dass es mit mindestens einem korrespondierenden Haltemittel eines Behälters für pharmazeutische Zubereitungen so zusammenwirken kann, dass ein Grundkörper des Behälters sicher an der Haltevorrichtung gehalten wird. Über die korrespondierenden Haltemittel sind also Haltekräfte von der Haltevorrichtung in den Grundkörper einleitbar, so dass dieser insbesondere gemeinsam mit der Haltevorrichtung handhabbar ist.

Bevorzugt wird eine Haltevorrichtung, bei der das Haltemittel so ausgebildet ist, dass eine unbeabsichtigte Relativbewegung zwischen einem in die Aufnahme eingesetzten Behälter und der Haltevorrichtung - in axialer, radialer und in Umfangsrichtung gesehen - vermieden wird. Der Behälter ist also bezüglich aller möglicher Freiheitsgrade sicher und stabil in der Aufnahme der Haltevorrichtung angeordnet. Eine unbeabsichtigte Relativbewegung kann nicht stattfinden, so dass kosmetische Defekte sicher vermieden werden. Der Behälter kann auch nicht aus der Haltevorrichtung herausfallen, wenn diese gewendet wird.

Bevorzugt wird eine Haltevorrichtung, bei der das Haltemittel so ausgebildet ist, dass es mit einem korrespondierenden Haltemittel an einem Grundkörper eines Behälters zusammenwirken kann. Umfasst also beispielsweise das Haltemittel des Behälters einen Vorsprung, umfasst bevorzugt das Haltemittel der Haltevorrichtung eine korrespondierende Ausnehmung und umgekehrt.

Bevorzugt wird außerdem ein Ausführungsbeispiel der Haltevorrichtung, die sich dadurch auszeichnet, dass das Haltemittel mindestens einen Vorsprung und/oder mindestens eine Ausnehmung aufweist.

Darüber hinaus wird bevorzugt eine Haltevorrichtung, die sich dadurch auszeichnet, dass das Haltemittel mindestens einen hakenförmigen Vorsprung umfasst.

Es wird eine Haltevorrichtung bevorzugt, die als Trägersystem für ein Haltesystem für pharmazeutische Behälter ausgebildet ist. Vorzugsweise sind an dem Trägersystem mehr als eine Aufnahme für mehr als einen Behälter vorgesehen. In diesem Fall wird auch eine Relativbewegung zwischen den Behältern durch die an den Aufnahmen vorgesehenen Haltemittel sicher vermieden. Es ergibt sich ein logistischer Vorteil dadurch, dass das Trägersystem mit den Behältern als Einheit gehandhabt werden kann. Es ist also nicht nötig, die einzelnen Behälter zu greifen. Sind Doppelkammersysteme in das Trägersystem eingesetzt, kann dieses zur Befüllung der beiden Kammern der Doppelkammersysteme ohne Weiteres gewendet werden, weil die Doppelkammersysteme nicht aus den Aufnahmen des Trägersystems herausfallen können. Es müssen demnach keine separaten Magazine mehr eingesetzt werden.

Es wird außerdem eine Haltevorrichtung bevorzugt, die mindestens eine Öffnung aufweist, durch welche eine Kammer eines in der Aufnahme angeordneten Behälters zugänglich ist.

Bevorzugt wird außerdem eine Haltevorrichtung, bei der im Bereich der Öffnung zwei sich - in Umfangsrichtung gesehen - gegenüberliegende, hakenförmige Vorsprünge vorgesehen sind.

Schließlich wird auch eine Haltevorrichtung bevorzugt, die als Mechanikteil für eine Injektionshilfe ausgebildet ist. Da in diesem Fall das Mechanikteil der Injektionshilfe eine Aufnahme aufweist, die ein Haltemittel umfasst, durch welches ein Behälter sicher gehalten wird, kann das ansonsten notwendige Gehäusevorderteil der Injektionshilfe entfallen. Insgesamt kann eine Injektionshilfe mit der Haltevorrichtung dann weniger Teile umfassen, so dass sie kostengünstiger und mit geringerem logistischem Aufwand herstellbar ist.

Schließlich wird ein Ausführungsbeispiel des Haltesystems bevorzugt, das sich dadurch auszeichnet, dass ein Wannenelement, in welchem das Trägersystem anordenbar ist, vorgesehen ist.

Weitere vorteilhafte Ausgestaltungen ergeben sich aus den Unteransprüchen.

Aufgabe der Erfindung ist es auch, ein Haltesystem zu schaffen, welches die beschriebenen Nachteile nicht aufweist.

Die Aufgabe wird gelöst, indem ein Haltesystem mit den Merkmalen des Anspruchs 4 geschaffen wird. Dieses zeichnet sich durch ein als Haltevorrichtung nach einem der Ansprüche 1 bis 3 ausgebildetes Trägersystem aus.

Weitere vorteilhafte Ausgestaltungen ergeben sich aus den Unteransprüchen.

Die Erfindung wird im Folgenden anhand der Figuren näher erläutert. Es zeigen:
- Figur 1: ein Ausführungsbeispiel eines Behälters für pharmazeutische Zubereitungen;
- Figur 2: ein Ausführungsbeispiel für ein Haltesystem und eine als Trägersystem ausgebildete Haltevorrichtung;
- Figur 3: eine Schnittansicht des Haltesystems und der Haltevorrichtung gemäß Figur 2;
- Figur 3A: eine Schnittansicht des Haltesystems mit einem abgewandelten Ausführungsbeispiel des Behälters;
- Figur 4: das Haltesystem gemäß Figur 2 mit mehreren in Aufnahmen des als Haltevorrichtung ausgebildeten Trägersystems angeordneten Behältern;
- Figur 5: ein Ausführungsbeispiel einer Injektionshilfe mit einer als Mechanikteil für die Injektionshilfe ausgebildeten Haltevorrichtung sowie einem Behälter in Explosionsdarstellung;
- Figur 6: die Injektionshilfe gemäß Figur 5 in zusammengesetztem Zustand;
- Figur 7: eine geschnittene Detailansicht der Injektionshilfe gemäß Figur 5;
- Figur 8: eine Schnittansicht der Injektionshilfe gemäß Figur 6;
- Figur 9: einen Behälter, eine als Fingerauflage ausgebildete Haltevorrichtung, eine Kolbenstange und einen Verschluss in Explosionsdarstellung;
- Figur 10: die Elemente gemäß Figur 9 in zusammengesetztem Zustand; und
- Figur 11: eine schematische Darstellung der Befüllung eines als Doppelkammersystem ausgebildeten Behälters unter Verwendung eines ein als Haltevorrichtung ausgebildetes Trägersystem umfassenden Haltesystems.

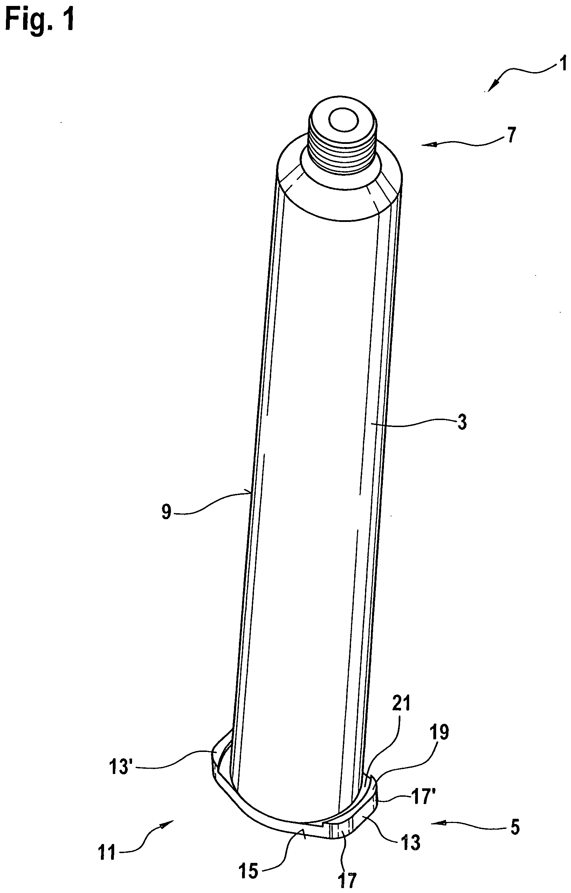

Figur 1 zeigt ein Ausführungsbeispiel eines Behälters 1 für pharmazeutische Zubereitungen. Der Behälter 1 ist hier als Karpule ausgebildet, wobei er als Ein- oder Doppelkammerkarpule ausgebildet sein kann. Bevorzugt ist der Behälter als Doppelkammerkarpule ausgebildet. Bei anderen, nicht dargestellten Ausführungsbeispielen ist der Behälter vorzugsweise als Spritze, insbesondere Ein- oder Doppelkammerspitze, oder als Phiole (Vial) ausgebildet.

Der Behälter 1 weist einen Grundkörper 3 auf, der vorzugsweise zylindrisch ausgebildet ist.

Ist der Behälter 1 als Spitze oder - wie hier dargestellt - als Karpule ausgebildet, umfasst er ein proximales Ende 5 und eine distales Ende 7. Der Grundkörper 3 weist eine Umfangsfläche 9 auf.

Es ist mindestens ein Haltemittel 11 vorzugsweise an dem Grundkörper 3 vorgesehen. Diese ist so ausgebildet, dass es mit einem korrespondierenden Haltemittel einer Haltevorrichtung für den Behälter zusammenwirken kann, um den Grundkörper sicher an der Haltevorrichtung zu halten. Dies bedeutet, dass über das Haltemittel 11 Haltekräfte in den Grundkörper 3 einleitbar sind, um diesen zu halten. Es ist dazu nicht erforderlich, dass das Haltemittel 11 unmittelbar an dem Grundkörper angeordnet oder mit diesem einstückig ausgebildet ist. Wesentlich ist, dass es so vorgesehen ist, dass die Haltekräfte in den Grundkörper 3 einleitbar sind.

Bei dem dargestellten, bevorzugten Ausführungsbeispiel ist das Haltemittel 11 allerdings unmittelbar an dem Grundkörper 3 vorgesehen. Es ist bevorzugt einstückig mit diesem ausgebildet.

Der Grundköper 3 kann bevorzugt Glas oder Kunststoff umfassen. Kosmetische Defekte sind besonders dann zu befürchten, wenn der Grundkörper 3 Kunststoff umfasst. Das Haltemittel 11 wird vorzugsweise unmittelbar an den Grundkörper 3 angeformt, wenn dieser Glas oder Kunststoff umfasst.

Das Haltemittel 11 umfasst bevorzugt mindestens einen Vorsprung und/oder mindestens eine Ausnehmung. Auf diese Weise ist es möglich, das korrespondierende Vorsprünge und/oder Ausnehmungen der Haltemittel des Behälters 1 einerseits und einer Haltevorrichtung für denselben andererseits vorzugsweise formschlüssig ineinander greifen, um den Behälter 1 an der Haltevorrichtung zu halten. Bevorzugt sind der Vorsprung beziehungsweise die Ausnehmung an der Umfangsfläche 9 vorgesehen.

Bevorzugt umfasst das Haltemittel 11 mindestens zwei Vorsprünge und/oder Ausnehmungen, die vorzugsweise auf einer Umfangslinie der Umfangsfläche 9 und besonders bevorzugt in gleichem Winkelabstand voneinander vorgesehen sind.

Es ist möglich, dass der Behälter 1 mindestens einen axialen Vorsprung und/oder mindestens eine axiale Ausnehmung umfasst.

Dabei spricht die Axialrichtung hier die Richtung der Längserstreckung des Behälters 1 an. Eine radiale Richtung ist eine Richtung, die senkrecht auf einer Längsachse des Behälters 1 steht. Mit einer Umfangsrichtung ist eine Richtung angesprochen, die sich um die Längsachse des Behälters 1 erstreckt.

Bei einem anderen bevorzugten Ausführungsbeispiel umfasst das Haltemittel 11 mindestens einen radialen Vorsprung und/oder mindestens eine radiale Ausnehmung. Diese sind ebenfalls bevorzugt an der Umfangsfläche 9 angeordnet.

Bei dem dargstellten Ausführungsbeispiel sind zwei sich - in radialer Richtung gesehen - gegenüberliegende radiale Vorsprünge 13, 13' vorgesehen. Bei einem anderen, nicht dargestellten Ausführungsbeispiel können entsprechend zwei radiale Ausnehmungen vorgesehen sein. Es ist auch möglich, sowohl mindestens einen radialen Vorsprung als auch mindestens eine radiale Ausnehmung vorzusehen beziehungsweise Vorsprünge und Ausnehmungen zu kombinieren. Bei wieder einem anderen Ausführungsbeispiel kann die Winkelteilung zwischen den Vorsprüngen und/oder Ausnehmungen ungleich sein. Die Elemente liegen sich dann nicht gegenüber.

Bei dem dargestellten Ausführungsbeispiel ist das Haltemittel 11 an dem proximalen Ende 5 des Grundkörpers 3 vorgesehen.

Die Vorsprünge 13, 13' sind bevorzugt identisch ausgebildet. Daher wird im Folgenden lediglich auf den Vorsprung 13 eingegangen, wobei vorausgesetzt wird, dass der Vorsprung 13' des dargestellten Ausführungsbeispiels identisch ausgebildet ist. Selbstverständlich ist es auch möglich, bei einem anderen Ausführungsbeispiel verschiedene Vorsprünge 13, 13' vorzusehen.

Der Vorsprung 13 weist an einer Umfangfläche 15 mindestens einen radialen Ansatz, hier zwei radiale Ansätze 17, 17' auf. Bei einem anderen Ausführungsbeispiel ist es möglich, das der Vorsprung 13 an seiner Umfangsfläche 15 mindestens eine Vertiefung aufweist. Umfasst das Haltemittel 11 bei wieder einem anderen Ausführungsbeispiel eine Ausnehmung, ist es möglich, dass diese an ihrer Umfangsfläche mindestens einen radialen Ansatz und/oder mindestens eine radiale Vertiefung aufweist.

Ein radialer Ansatz 17, 17' oder eine entsprechende radiale Vertiefung ist vorzugsweise vorgesehen, um die Reibung des Haltemittels 11 an einer korrespondierenden Fläche des Haltemittels einer Haltevorrichtung zu erhöhen. Es ist aber auch möglich, dass der Ansatz 17, 17' oder die entsprechende Vertiefung mit einer Vertiefung oder einem entsprechenden Ansatz des korrespondierenden Haltemittels so zusammenwirken, dass quasi eine Verrastung des Behälters 1 in der Haltevorrichtung resultiert. Insoweit kann der Ansatz 17, 17' oder ein entsprechender Ansatz an dem korrespondierenden Haltemittel auch als Rastelement bezeichnet werden.

Der Vorsprung 13 weist mindestens einen, hier genau einen axialen Ansatz 19 auf. Hierdurch wird - in radialer Richtung gesehen - zwischen dem axialen Ansatz 19 und der Umfangsfläche 9 eine Vertiefung oder Nut 21 ausgebildet. Bei einem anderen Ausführungsbeispiel kann auch anstelle des axialen Ansatzes 19 oder zusätzlich zu diesem mindestens eine axiale Vertiefung, vorzugsweise eine Nut an dem Vorsprung 13 vorgesehen sein. Umfasst das Haltemittel 11 bei wieder einem anderem, nicht dargestellten Ausführungsbeispiel mindestens eine Ausnehmung, ist es möglich, dass diese mindestens einen axialen Ansatz und/oder mindestens eine axiale Vertiefung, vorzugsweise eine Nut, aufweist.

Bei dem dargestellten Ausführungsbeispiel ist der Vorsprung 13 - im nicht dargestellten Querschnitt gesehen - quasi hakenförmig ausgebildet. Dies ergibt sich dadurch, dass er einerseits den axialen Ansatz 19 und andererseits die axiale Vertiefung 21 umfasst. Der axiale Ansatz 19 kann von einem korrespondierenden Haltemittel hintergriffen werden, so dass eine formschlüssige Verbindung resultiert.

Bevorzugt ist das Haltemittel 11 so ausgebildet, dass es mit einem korrespondierenden Haltemittel nach Art eines Bajonettverschlusses zusammenwirken kann. Typischerweise umfasst ein Bajonettverschluss eine Steck-Dreh-Mechanik, bei dem mindestens ein Vorsprung an einem Teil zunächst in Längsrichtung in eine an dem anderen Teil vorgesehene erste Ausnehmung eingeführt wird, wobei sich eine zweite Ausnehmung im Wesentlichen senkrecht an die erste Ausnehmung anschließt, allerdings - in Einführrichtung des Vorsprungs gesehen - nach vorne versetzt. Der Vorsprung muss zunächst in einer Steckbewegung eine gewisse Strecke entlang der ersten Ausnehmung zurücklegen, bevor er anschließend mit Hilfe einer Drehbewegung in die zweite Ausnehmung eingedreht werden kann. Da sich die zweite Ausnehmung im Wesentlichen senkrecht zu der ersten Ausnehmung erstreckt, können dann die beiden Teile nicht mehr durch eine rein translatorische Relativbewegung getrennt werden. Üblicherweise wird der Bajonettverschluss - in Längsrichtung gesehen - vorgespannt, so dass der Vorsprung gegen die Ausnehmung gedrängt wird und sich hier eine gewisse Haftreibung ergibt.

Die zweite Ausnehmung kann auch schräg zu der ersten Ausnehmung verlaufen, wobei sie dann eine Fläche umfasst, die - entgegen der Einführrichtung gesehen - abfällt. Ist der Verschluss - in Längsrichtung gesehen - vorgespannt, muss zunächst eine bestimmte Kraft aufgebracht werden, um den Vorsprung in die zweite Ausnehmung einführen zu können. Die auf den Vorsprung in der Rastposition wirkende Vorspannkraft ist dann geringer als diese Kraft. Um die beiden Teile relativ zueinander zu entriegeln, muss wieder die entsprechende größere Kraft aufgebracht werden. Der Vorsprung sitzt daher in der Rastposition quasi in einem Potentialminimum.

Der Begriff Rastposition bezeichnet hier im folgenden allgemein die Position des Behälters 1 an einer Haltevorrichtung, in welcher dieser sicher und fest an derselben angeordnet ist. Der Begriff meint nicht, dass notwendigerweise eine Verrastung zwischen den korrespondierenden Halteelementen vorliegen muss.

Der mindestens eine Vorsprung und/oder die mindestens eine Ausnehmung des Haltemittels 11 weist/weisen bevorzugt mindestens eine abfallende Fläche auf. Diese kann als abfallende Fläche eines Bajonettverschlusses - wie soeben beschrieben - ausgebildet sein.

Es ist aber auch möglich, dass die mindestens eine abfallende Fläche bevorzugt als Einführschräge zur Befestigung des Haltemittels an einem korrespondierenden Haltemittel einer Haltevorrichtung vorgesehen ist. Eine solche Einführschräge kann beispielsweise dazu dienen, ein elastisch gelagertes Rastelement beim Einführen des Haltemittels 11 zu verdrängen, wobei das elastische Rastelement dann in eine im Bereich der Einführschräge angeordnete Hinterschneidung einrastet, wenn das Haltemittel 11 weit genug in das korrespondierende Haltemittel eingeführt wurde. Der Behälter 1 ist dann sicher an der Haltevorrichtung verrastet.

Die Einführschräge kann aber bevorzugt auch dazu vorgesehen sein, eine beim Verbinden der korrespondierenden Haltemittel fortschreitende Pressung beziehungsweise einen sich verstärkenden Reibschluss zu bewirken. Insbesondere ist es möglich, dass letztlich eine Selbsthemmung zwischen den korrespondierenden Haltemitteln vorliegt, so dass diese sicher aneinander halten.

Generell ist es möglich, dass mindestens ein Vorsprung und/oder mindestens eine Ausnehmung des Haltemittels 11 mindestens eine abfallende Fläche aufweist/aufweisen, wobei die mindestens eine abfallende Fläche vorzugsweise als Einführschräge zur Befestigung des Haltemittels an einem korrespondierenden Haltemittel einer Haltevorrichtung vorgesehen ist.

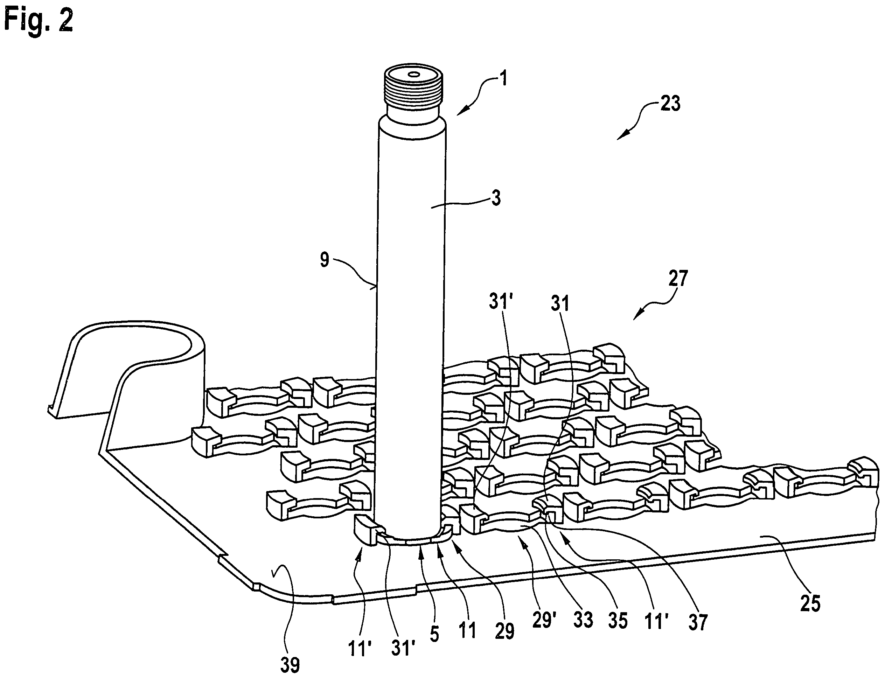

Figur 2 zeigt ein Ausführungsbeispiel eines Haltesystems 23 für Behälter für pharmazeutische Zubereitungen mit einer als Trägersystem 25 ausgebildeten Haltevorrichtung 27. Gleiche und funktionsgleiche Elemente sind mit gleichen Bezugszeichen versehen, so dass insofern auf die vorangegangene Beschreibung verwiesen wird. In einer Aufnahme 29 der Haltevorrichtung 27 ist ein Behälter 1 angeordnet.

Das hier dargestellte Trägersystem 25, welches als Haltevorrichtung 27 ausgebildet ist, wird vorzugsweise in Zusammenhang mit einem Haltesystem 23 verwendet, welches außerdem ein nicht dargestelltes Wannenelement umfasst, in welchem das Trägersystem 25 anordenbar ist. Solche Haltesysteme, welche dem Transport, insbesondere der Auslieferung und der Handhabung einer bestimmten Anzahl von Behältern 1 dienen, werden üblicherweise auch als Nest/Tub-Konfiguration bezeichnet. Das sogenannte Tub ist ein Wannenelement, in welches das als Nest ausgebildete Trägersystem 27 eingesetzt werden kann. Vorzugsweise wird das Tub durch eine Folie verschlossen. Auf diese Weise ist es insbesondere möglich, eine bestimmte Anzahl von Behältern 1 in vorsterilisiertem Zustand zu transportieren und steril zu halten, so dass die Behälter 1 in dem Tub in einen Reinraum zur Abfüllung eingebracht werden können, ohne dass sie erneut sterilisiert werden müssen. Mit Hilfe des Nests beziehungsweise des Trägersystems 25 können die Behälter 1 gehandhabt werden, ohne dass sie einzeln gegriffen werden müssen.

Vorzugsweise orientieren sich die Maße des Trägersystems 25 und des nicht dargestellten Wannenelements an standardisierten Verpackungsformen. Hierdurch ist das Haltesystem 23 in genormten Abfülllinien einsetzbar. Bekannte beziehungsweise vorhandene Maschinen- und Prozesstechnik ist verwendbar, so dass keine oder nur geringfügige Anpassungen vorhandener Anlageteile oder bekannter Prozesse für die Verwendung des Haltesystems 23 nötig sind.

Die in Figur 2 dargestellte Haltevorrichtung 25 umfasst mehrere Aufnahmen 29 für Behälter 1, die bevorzugt identisch ausgebildet sind. Es wird daher im Folgenden lediglich auf die Aufnahme 29 beziehungsweise auf die daneben angeordnete Aufnahme 29' Bezug genommen. Selbstverständlich ist es auch möglich, an einer Haltevorrichtung 27 verschiedene Aufnahmen vorzusehen.

Die Aufnahme 29, 29' umfasst ein Haltemittel 11', welches so ausgebildet ist, dass es mit dem korrespondierenden Haltemittel 11 des Behälters 1 so zusammenwirkt, dass der Grundkörper 3 sicher an der Haltevorrichtung 27 gehalten wird.

Es ist möglich, dass die Haltevorrichtung 27 mindestens eine Aufnahme umfasst, die einen Durchmesser aufweist, der geringfügig kleiner ist als der Außendurchmesser des Grundkörpers 3. Ist dann entweder die Haltevorrichtung 27 zumindest im Bereich der Aufnahme 29 oder der Grundkörper 3 hinreichend elastisch ausgebildet, können die beiden Elemente durch Einführen des Behälters 1 in die Aufnahme 29 verbunden werden, wobei sie reibschlüssig aneinander halten. Hierbei können jedoch kosmetische Defekte insbesondere im Bereich der Umfangsfläche 9 auftreten.

Es wird daher ein Ausführungsbeispiel der Haltevorrichtung 27 bevorzugt, bei dem ein Formschluss zwischen korrespondierenden Haltemittel bewirkt wird.

Dabei ist es möglich, dass die Haltevorrichtung 27 elastische Vorsprünge oder quasi eine elastische Ringnut aufweist, in die ein Flansch eines Behälters 1, der in bekannter Weise vorzugsweise an dessen proximalem Ende 5 vorgesehen ist, eingeklipst wird.

Bevorzugt weist jedoch das Haltemittel 11 mindestens zwei radiale Vorsprünge 13, 13' auf.

Das Haltemittel 11' der Haltevorrichtung 27 ist bevorzugt so ausgebildet, dass eine unbeabsichtigte Relativbewegung zwischen einem in die Aufnahme 29, 29' eingesetzten Behälter 1 und der Haltevorrichtung 27 - in axialer, radialer und in Umfangsrichtung gesehen - vermieden wird. Der Behälter 1 wird also derart in der Haltevorrichtung 27 gehalten, dass er in keinem dieser Freiheitsgrade unbeabsichtigt beweglich ist. Es wird also eine besonders stabile Halterung vorgesehen. Der Behälter 1 kann sich insbesondere nicht unbeabsichtigt relativ zu der Haltevorrichtung 27 drehen oder aus dieser herausfallen. Ebenso kommt der Behälter 1 nicht unbeabsichtigt mit den in benachbarten Aufnahmen angeordneten Behältern 1 in Kontakt. Kosmetische Defekte werden so vermieden.

Das Haltemittels 11' ist bevorzugt so ausgebildet, dass es mit dem korrespondierenden Haltemittel 11 des Behälters 1 zusammenwirkt. Dies bedeutet, dass die in Zusammenhang mit dem Haltemittel 11 des Behälters 1 beschriebenen Merkmale auch auf das Haltemittel 11' der Haltevorrichtung 27 zutreffen. Insbesondere ist das Haltemittel 11' vorzugsweise komplementär zu dem Haltemittel 11 ausgebildet. Es kann also entsprechende Vorsprünge, Ausnehmungen, Ansätze und/oder Vertiefungen aufweisen, die bevorzugt komplementär zu den entsprechenden Elementen des Haltemittels 11 ausgebildet sind. Auf eine allgemeine Beschreibung des Haltemittels 11' wird daher verzichtet und insoweit auf die Beschreibung des Haltemittels 11 verwiesen. Im Folgenden wird allerdings konkret auf das dargestellte Ausführungsbeispiel der Haltevorrichtung 27 und des Haltemittels 11' eingegangen.

Das Haltemittel 11' umfasst mindestens einen Vorsprung und/oder mindestens eine Ausnehmung, bevorzugt mindestens einen hakenförmigen Vorsprung, hier konkret zwei hakenförmige Vorsprünge 31, 31'. Die Haltevorrichtung 27 weist außerdem im Bereich der Ausnehmung 29' eine Öffnung 33 auf. Durch diese ist eine Kammer eines in der Aufnahme 29' angeordneten Behälters 1 zugänglich. Insbesondere ist es vorzugsweise möglich, die Kammer durch die Öffnung 33 zu befüllen. Ganz besonders bevorzugt kann auch ein Stopfen durch die Öffnung 33 in den Behälter 1 eingeführt werden. Dies kann beispielsweise ein Mittelstopfen eines Doppelkammersystems oder ein Endstopfen einer Spritze, Karpule oder eines Doppelkammersystems sein. Die Öffnung 33 muss hierzu einen Durchmesser aufweisen, der mindestens genauso groß, vorzugsweise geringfügig größer ist als der Innendurchmesser des Behälters 1 im Bereich der zu befüllenden Kammer beziehungsweise in dem Bereich, in den der Stopfen eingesetzt werden soll. Bevorzugt ist die Öffnung 33 allerdings bezüglich ihres Durchmessers so bemessen, dass sich der Behälter 1 noch zumindest mit einem Bereich der Wandung des Grundkörpers 3 an der Haltevorrichtung 27 abstützen kann. Demgemäß ist der Durchmesser der Öffnung 33 bevorzugt kleiner als ein Außendurchmesser des Grundkörpers 3 in dem Bereich, mit dem der Grundkörper 3 an der Haltevorrichtung 27 anliegt.

Die hakenförmigen Vorsprünge 31, 31' sind bei dem dargestellten Ausführungsbeispiel - in radialer Richtung der Öffnung 33 gesehen - gegenüberliegend angeordnet. Da sie vorzugsweise identisch ausgebildet sind, wird im Folgenden nur der Vorsprung 31 näher beschrieben.

Dieser weist einen axialen Ansatz 35 und eine entsprechende axiale Vertiefung 37 auf. Hierdurch wird im Wesentlichen die Hakenform des Vorsprungs 31 gebildet. In der Rastposition des Behälters 1 greift der Vorsprung 13 mit dem Ansatz 19 in die Vertiefung 37 ein, während zugleich der Ansatz 35 in die Vertiefung 21 eingreift. Es entsteht so ein Formschluss, und der Behälter 1 wird sowohl in axialer als auch in radialer Richtung sicher an der Haltevorrichtung 27 gehalten.

Die an dem Vorsprung 13 vorgesehenen radialen Ansätze 17, 17' sind bei dem dargestellten Ausführungsbeispiel vorgesehen, um die Reibung zwischen dem Vorsprung 13 und dem Vorsprung 31 zu erhöhen, so dass insbesondere auch ein sicherer Halt des Behälters 1 an der Haltevorrichtung 27 - in Umfangsrichtung gesehen - gewährleistet ist.

Der Vorsprung 13' wirkt in entsprechender Weise mit dem Vorsprung 31' zusammen.

Der Behälter 1 kann bei dem dargestellten Ausführungsbeispiel in seine Rastposition geschwenkt werden. Dazu wird er zunächst um etwa 90° zu seiner dargestellten Position versetzt in der Aufnahme 29 angeordnet und dann um etwa 90° geschwenkt, wodurch die Vorsprünge 13, 13' und 31, 31' miteinander in Eingriff kommen, so dass der Behälter 1 letztlich in seiner Rastposition angeordnet ist. Dabei stützen sich die Vorsprünge 13, 13' an einer Anlagefläche 39 ab.

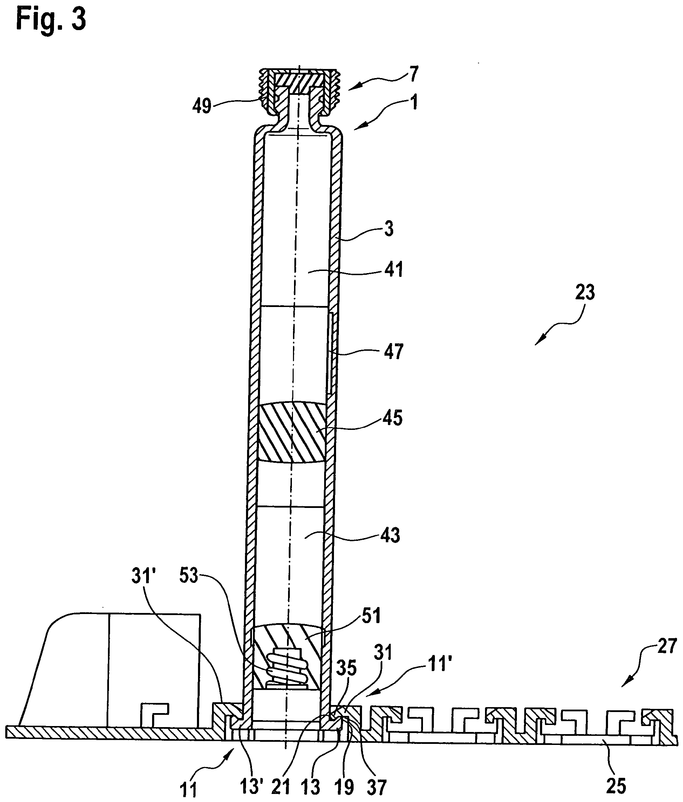

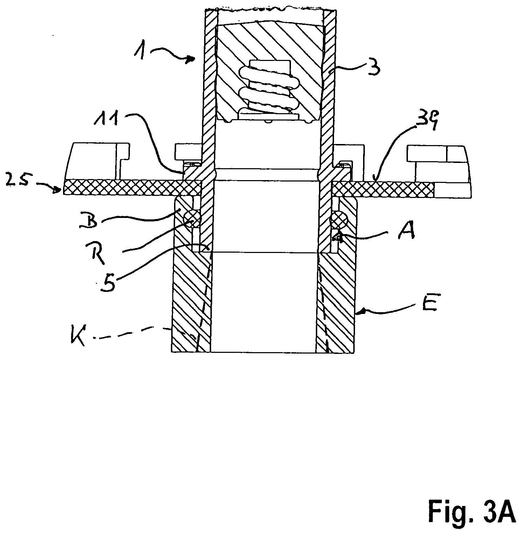

Figur 3 zeigt eine Schnittansicht des Haltesystems gemäß Figur 2. Gleiche und funktionsgleiche Elemente sind mit gleichen Bezugszeichen versehen, so dass insoweit auf die vorangegangene Beschreibung verwiesen wird. Auch in Figur 3 ist dargestellt, dass der Vorsprung 13 mit dem Vorsprung 31 so zusammenwirkt, dass der Ansatz 19 in die Vertiefung 37 eingreift, während der Ansatz 35 in die Vertiefung 21 eingreift. Entsprechendes gilt für den Vorsprung 13' und den Vorsprung 31'.

Die Vorsprünge 31, 31' liegen sich entlang des Umfangs der Öffnung 33 gegenüber.

Der Behälter 1 ist bei dem in Figur 3 dargestellten Ausführungsbeispiel als Doppelkammersystem ausgebildet. Er umfasst eine distale Kammer 41 und eine proximale Kammer 43, die durch einen Mittelstopfen 45 voneinander getrennt sind. Zur Aktivierung des Doppelkammersystems kann der Mittelstopfen 45 in bekannter Weise in den Bereich eines Bypasses 47 verlagert werden, um eine Fluidverbindung zwischen der distalen Kammer 41 und der proximalen Kammer 43 herzustellen.

Der Mittelstopfen 45 kann durch die Öffnung 33, somit durch die Haltevorrichtung 27 beziehungsweise das Trägersystem 25 hindurch in das Innere des Grundkörpers 3 eingebracht werden.

Das distale Ende 7 des Behälters 1 ist mit einem Verschluss 49 verschlossen. Wird die in der distalen Kammer 41 vorliegende pharmazeutische Zubereitung nicht gefriergetrocknet, ist es möglich, den Behälter 1 vollständig über die Öffnung 33 zu beschicken. Der Behälter wird dann zunächst mit dem Verschluss 49 verschlossen. Dann wird die distale Kammer 41 befüllt. Danach wird der Mittelstopfen 45 gesetzt. Anschließend wird die proximale Kammer 43 befüllt. Schließlich wird ein Endstopfen 51 gesetzt. Dieser weist bevorzugt ein Verbindungsmittel zum Herstellen einer Verbindung mit einer Kolbenstange, hier ein Gewinde 53 auf.

Soll allerdings die in der distalen Kammer 41 angeordnete pharmazeutische Zubereitung gefriergetrocknet werden, ist eine andere Abfolge von Befüllungsschritten nötig. Hierbei muss der Behälter 1 gewendet werden. Bei dem dargestellten Ausführungsbeispiel ist dies möglich, indem die Haltevorrichtung 27 beziehungsweise das Trägersystem 25 gewendet wird. Da der Behälter 1 sicher an diesem angeordnet ist, kann er dabei nicht unbeabsichtigt von dem Trägersystem 25 getrennt werden oder herausfallen. Auf das entsprechende Befüllen eines Doppelkammersystems wird im Folgenden noch näher eingegangen.

Bei den dargestellten Ausführungsbeispielen weist der Behälter 1 das mindestens ein Haltemittel 11 an seinem proximalen Ende 5 auf. Bei anderen Ausführungsbeispielen ist es auch möglich, dass das mindestens ein Haltemittel 11 in einem anderen Bereich des Behälters 1 angeordnet ist. Beispielsweise könnte ein Haltemittel 11 mehr oder weniger mittig an dem Behälter 1 angeordnet sein. Entsprechend wäre dann auch die Haltevorrichtung 27 in dem entsprechenden mittigen Bereich des Behälters 3 angeordnet. Es ist auch möglich, mindestens ein Haltemittel 11 an dem distalen Ende 7 des Behälters 1 vorzusehen.

Es zeigt sich, dass bei den dargestellten Ausführungsbeispielen der Behälter 1 im Wesentlichen durch eine Dreh- beziehungsweise Schwenkbewegung mit der Haltevorrichtung 27 verrastet wird. Dabei können kosmetische Defekte an dem Behälter 1 auftreten, wenn dieser mit einem Werkzeug gegriffen wird, welches das zur Verrastung nötige Drehmoment reibschlüssig aufbringt. Daher umfasst der Behälter 1 bevorzugt ein Verbindungselement, das so ausgebildet ist, dass ein Werkzeug formschlüssig aufgenommen werden kann, um eine Drehmomentübertragung von dem Werkzeug auf den Grundkörper zu bewirken. Vorzugsweise kann eine Innenwandung des Grundkörpers 3 im Bereich des proximalen Endes 5 so ausgebildet sein, dass ein Werkzeug formschlüssig eingreifen kann. Beispielsweise kann hier eine Vierkantaufnahme ausgebildet sein. Auch andere Geometrien wie beispielsweise eine Sechskantaufnahme sind denkbar. Auch eine Mehrkant- oder ähnliche Geometrie an der Umfangsfläche 9 zum Angreifen eines entsprechenden Schlüssels ist möglich. Es ist auch möglich, mindestens eine Erhöhung und/oder Vertiefung an einem stirnseitigen Ende des Behälters 1, vorzugsweise also im Bereich des proximalen Endes 5 oder des distalen Endes 7 vorzusehen, mit denen ein Werkzeug zusammenwirken kann, um das zur Verrastung nötige Drehmoment aufzubringen.

Bevorzugt kann auch eine Steckverbindung vorgesehen sein, bei der der Behälter 1 ins Längsrichtung in eine entsprechend ausgebildete Aufnahme der Haltevorrichtung 27 eingeführt wird.

Bevorzugt weist wenigstens eines der Haltemittel 11, 11' eine abfallende Fläche auf. Bei dem dargestellten Ausführungsbeispiel ist es möglich, dass beispielsweise der Grund der Vertiefung 37 oder der Grund der Vertiefung 21 als abfallende Fläche ausgebildet ist. Diese fällt dann vorzugsweise in Umfangsrichtung gesehen ab. Dementsprechend hat die Vertiefung, welche die abfallende Fläche aufweist, - in Umfangsrichtung gesehen - eine veränderliche Tiefe. Vorzugsweise kommt zunächst der tiefste Bereich der Vertiefung mit dem in sie eingreifenden Ansatz in Eingriff. Bei einer weiteren Bewegung in Richtung auf die Rastposition hin verringert sich die Tiefe der Vertiefung, bis der entsprechende Ansatz an deren Grund anliegt. Vorzugsweise weist wenigstens eins der beiden miteinander zu verbindenden Haltemittel 11, 11' eine gewisse Elastizität auf, so dass eine weitere Bewegung in Richtung auf die Rastposition hin möglich ist, wobei wenigstens eines der beiden Elemente zumindest geringfügig deformiert wird. Hierdurch entsteht eine Vorspannkraft, die einer unbeabsichtigten Trennung der Verbindung entgegenwirkt. Die mindestens eine abfallende Fläche kann mit einem geeigneten Winkel abfallen, so dass in diesem Bereich Selbsthemmung auftritt.

Eine entsprechend abfallende Fläche kann auch an dem Ansatz 35 oder dem Ansatz 19 vorgesehen sein. Auch ist es möglich, an anders als in den dargestellten Ausführungsbeispielen ausgebildeten Haltemitteln abfallende Fläche auszubilden.

Es ist möglich, vorzugsweise mindestens ein elastisch verlagerbares Rastelement, beispielsweise einen elastisch gelagerten Stift oder eine Rastnase beziehungsweise Rastzunge vorzusehen, das bevorzugt durch eine abfallende Fläche beim Ineingriffbringen der korrespondierenden Haltemittel zunächst zurückfedert oder zurückschwenkt, um schließlich in der Rastposition in eine Hinterschneidung einzurasten, die - in Einführrichtung gesehen - hinter der als Einführschräge wirkenden abfallenden Fläche angeordnet ist. Je nach Art der Verbindung kann die Einführrichtung mit der Längsrichtung (Steckverbindung) oder mit einer Umfangsrichtung des Behälters 1 (Dreh- oder Schwenkverbindung) zusammenfallen.

Figur 3A zeigt eine vergrößerte Schnittansicht des Haltesystems gemäß Figur 3. Gleiche und funktionsgleiche Elemente sind mit gleichen Bezugszeichen versehen, sodass insoweit auf die vorangegangene Beschreibung verwiesen wird.

Figur 3A zeigt ein abgewandeltes Ausführungsbeispiel des Behälters 1, in dessen proximales Ende 5 ein Endstopfen 51 eingesetzt ist. Der Behälter 1 unterscheidet sich von den in Figur 3 dargestellten dadurch, dass die Haltemittel 11 nicht unmittelbar an dem proximalen Ende 5 des Behälters vorgesehen sind, sondern in einem Abstand dazu. Wird das Haltemittel 11 des Behälters 1 mit einem Haltemittel 11' einer Aufnahme 29 einer Haltevorrichtung 27 gekoppelt, so ragt ein proximaler Abschnitt A des Grundkörpers 3 des Behälters 1 aus dem Trägersystem 25. Figur 3A zeigt, dass oben über das Trägersystem 25 der Grundkörper 3 hervorsteht und mit seinem Haltemittel 11 auf der Anlagefläche 39 des Trägersystems 25 aufliegt. Auf der gegenüberliegenden Seite der Anlagefläche 39 ragt der Abschnitt A aus dem Trägersystem 25. Vorzugsweise ist der Abschnitt A einstückig mit dem Grundkörper 3 des Behälters 1 ausgebildet, wie dies in Figur 3A dargestellt ist.

Figur 3A ist zu entnehmen, dass auf den Abschnitt A vorzugsweise eine Einführhilfe E aufsetzbar ist, deren Innendurchmesser dem Innendurchmesser des Grundkörpers 3 des Behälters 1 entspricht. Die Einführhilfe E ist mit einem Befestigungsflansch versehen, der den Abschnitt A von außen umgreift, sodass die Einführhilfe E sicher an dem Abschnitt A gehalten wird.

Bei dem hier dargestellten Ausführungsbeispiel ist zwischen der Außenfläche des Abschnitts A und der Innenfläche des Befestigungsflansches B ein hier vorzugsweise als O-Ring R ausgebildetes Verbindungselement vorgesehen.

Bei dem in Figur 3A dargestellten Ausführungsbeispiel ist die Einführhilfe E zylindrisch ausgebildet. Ebenso ist der Befestigungsflansch B zylindrisch, also ringförmig ausgestaltet. Es ist aber sehr wohl denkbar, dass dieser Befestigungsflansch B nicht als geschlossener Ring ausgebildet ist, sondern eine Anzahl von einzelnen Befestigungsarmen aufweist, über welche die Einführhilfe E an dem Abschnitt A des Grundkörpers 3 gehalten wird.

Die ringförmige Ausgestaltung des Befestigungsflansches B wird besonders in Verbindung mit dem O-Ring R bevorzugt. Auf diese Weise ist es nämlich möglich, die Einführhilfe E dicht an den proximalen Abschnitt A des Grundkörpers 3 des Behälters 1 aufzusetzen. Eine Stopfensetz-Vorrichtung kann dann so an der Einführhilfe E angesetzt werden, dass ein Vakuum in Inneren des Behälters aufgebaut wird, bevor dann ein Stopfen eingesetzt wird. Der O-Ring R dichtet dabei also die Einführhilfe E so gegenüber dem Abschnitt A ab, dass das Vakuum verlustfrei im Behälter 1 aufgebaut werden kann.

Beim Stopfen-Setzen kann die Einführhilfe E auch als Orientierungshilfe für die Stopfensetz-Vorrichtung dienen. Damit ist eine optimale Ausrichtung dieser Vorrichtung gegenüber dem mit einem Stopfen zu versehenden Behälter 1 möglich.

Nach allem ist aus Figur 3A ersichtlich, dass bei dem abgewandelten Ausführungsbeispiel des Behälters 1 dessen Haltemittel 11, wie bei dem zuerst beschriebenen Ausführungsbeispiel des Behälters 1, auf der Anlagefläche 39 des Trägersystems 25 aufliegen. Der Grundkörper 3 des Behälters 1 ragt allerdings bei dem Ausführungsbeispiel gemäß Figur 3A durch das Trägersystem 25 hindurch, sodass ein Abschnitt A des Grundkörpers 3 über die der Anlagefläche 39 gegenüberliegende Seite des Trägersystems 25 hinausragt. Es ist damit möglich, auf den Abschnitt A eine Einführhilfe E aufzusetzen, welche das Einbringen eines oder mehrerer Stopfen in das Innere des Behälters 1 erleichtern. Das sogenannte Stopfen-Setzen wird dadurch wesentlich verbessert, damit auch der Abfüllprozess des Behälters 1.

Besonders vorteilhaft ist es dabei, dass die Innenfläche der Einführhilfe E in die Innenfläche des Abschnitts A des Grundkörpers 3 des Behälters 1 übergeht, sodass ein in die Einführhilfe E eingeführter Stopfen sehr leicht in den Grundkörper 3 des Behälters 1 überführt werden kann.

Es zeigt sich im Übrigen, dass der über das Trägersystem 25 hinausragende Abschnitt A bereits das Stopfen-Setzen vereinfacht, auch wenn keine Einführhilfe E der in Figur 3A beschriebenen Art vorgesehen ist. Eine Stopfensetz-Vorrichtung kann also unmittelbar an dem Abschnitt A angesetzt werden. Durch diese Einführhilfe E können jedoch das Stopfen-Setzen besonders vereinfacht und der Abfüllprozess damit verbessert werden.

Aus den Erläuterungen zu Figur 3A ist ohne Weiteres ersichtlich, dass die Einführhilfe E, die hier zylindrisch ausgebildet ist und einen zylindrischen Innenraum aufweist, auch mit einem konischen Innenraum versehen werden kann, der an dem dem Abschnitt A abgewandten Ende der Einführhilfe E einen größeren Innendurchmesser aufweist, als an dem dem Abschnitt A zugeordneten Ende. Das freie Ende der Einführhilfe E hat also damit einen größeren Innendurchmesser als der Grundkörper 3 und der Abschnitt A. Ein Stopfen kann somit sehr leicht in das freie Ende der Einführhilfe E eingebracht und dann in das Innere des Grundkörpers 3 überführt werden, insbesondere dann, wenn im Übergangsbereich des Innenraums der Einführhilfe E zum Innenraum des Abschnitts A gleiche Innendruchmesser vorgesehen sind, sodass hier ein stoßfreier Übergang von der Einführhilfe E zum Abschnitt A vorgesehen ist.

In Figur 3A ist ein konischer Innenraum der Einführhilfe E durch eine gestrichelte Linie K angedeutet. Der Neigungswinkel dieser Linie K gegenüber einer zylindrischen Innenfläche kann an verschiedene Stopfen angepasst werden, die in Zusammenhang mit dem Behälter 1 verwendet werden. Je mehr die Stopfen beim Einführen in den Behälter 1 komprimiert werden müssen, umso größer wird vorzugsweise der Neigungswinkel des konischen Innenbereiches gewählt. Das heißt also, umso größer ist der Neigungswinkel der Linie K gegenüber der hier zylindrisch dargestellten Innenfläche der Einführhilfe E, umso weiter öffnet sich damit der Innenraum der Einführhilfe in Richtung auf deren freies Ende.

Aus dem oben Gesagten wird ohne Weiteres deutlich, dass der konische Innenbereich in der Einführhilfe E sich nicht über die gesamte Länge der Einführhilfe E von deren freien Ende bis zum Ende des Abschnitts A erstrecken muss. Es reicht, wenn ein konischer Bereich unmittelbar am freien Ende der Einführhilfe E ausgebildet wird, um das Einführen eines Stopfens zu erleichtern.

Überdies ist es möglich, den Rand des Innenraums im Bereich des freien Endes der Einführhilfe E mit einem Radius auszubilden, um das Einführen eines Stopfens zu erleichtern.

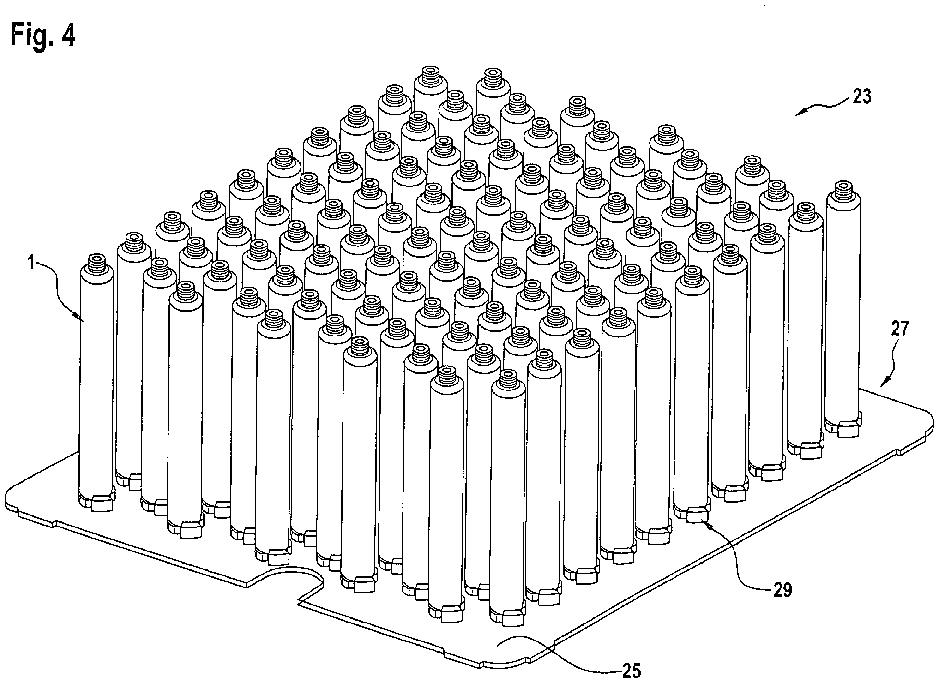

Figur 4 zeigt das Haltesystem gemäß Figur 2, wobei das komplette Trägersystem 25 dargestellt ist. Gleiche und funktionsgleiche Elemente sind mit gleichen Bezugszeichen versehen, so dass insofern auf die vorangegangenen Ausführungen verwiesen wird. Bei dem dargestellten Ausführungsbeispiel ist in jeder der Aufnahmen, von denen hier nur die Aufnahme 29 exemplarisch mit einem Bezugszeichen versehen ist, ein Behälter 1 angeordnet. Es wird deutlich, dass es sich bei dem dargestellten Ausführungsbeispiel des Trägersystems 25 um ein Nest einer Nest-/Tub-Konfiguration handelt, in der eine vorbestimmte Anzahl von Behältern 1 vorsterilisiert ausgeliefert und gehandhabt werden kann. Es zeigt sich auch, dass zum Befüllen der Behälter 1 lediglich das Trägersystem 25 gegriffen werden muss. Es ist also nicht nötig, die einzelnen Behälter 1 zu greifen und in separaten Vorrichtungen anzuordnen. Da die Behälter 1 sicher und fest an dem Trägersystem 25 gehalten sind, kann der ganze Befüllungsprozess auf sehr einfache und insbesondere auch automatisierte Weise durchgeführt werden, indem die Behälter simultan über das Trägersystem gegriffen beziehungsweise gehandhabt werden.

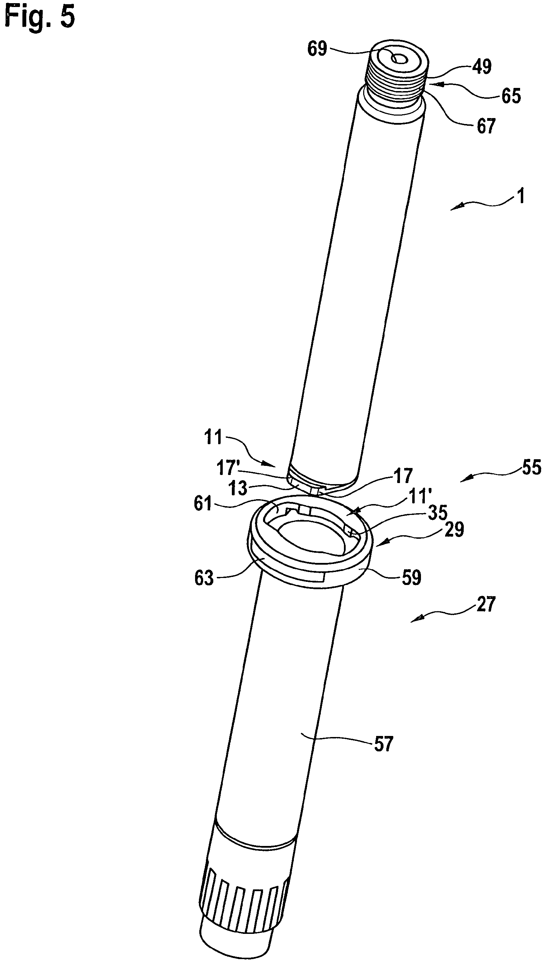

Figur 5 zeigt eine Injektionshilfe 55 in teilweise zerlegtem Zustand. Die Haltevorrichtung 27 ist hier als Mechanikteil 57 für die Injektionshilfe 55 ausgebildet. Gleiche und funktionsgleiche Elemente sind mit gleichen Bezugszeichen versehen, so dass insofern auf die vorangegangene Beschreibung verwiesen wird. Das Mechanikteil 57, welches als Haltevorrichtung 27 ausgebildet ist, weist eine Aufnahme 29 auf. Diese umfasst ein Haltemittel 11', welches hier im Wesentlichen identisch ausgebildet ist zu dem Haltemittel 11' der Haltevorrichtung 27 der Figuren 1 bis 4. Allerdings ist bei dem dargestellten Ausführungsbeispiel statt der beiden Vorsprünge 31, 31' ein Haltering 59 vorgesehen, an dem der Ansatz 35 und die hier nicht dargestellte Vertiefung 37 sowie die ebenfalls nicht dargestellten gegenüberliegenden Elemente, nämlich der entsprechende Ansatz und die entsprechende Vertiefung, angeordnet sind. Der Ring 59 weist außerdem eine stirnseitige Öffnung 61 auf, durch welche der Behälter 1 mit seinem Haltemittel 11 in die Aufnahme 29 einsetzbar ist. Dabei weist die Öffnung 61 eine zu dem Haltemittel 11 komplementäre Form auf, so dass der Behälter 1 nur in einer Position, die in etwa 90° um die Längsachse relativ zu der dargestellten Rastposition geschwenkt ist, einsetzbar ist.

Im Übrigen kann eine Verbindung der Haltemittel 11, 11' in gleicher Weise bewirkt werden, wie dies in Hinblick auf die bereits beschriebenen Ausführungsbeispiele der Fall ist. Auch Abweichungen von der dargestellten Konfiguration in Hinblick auf andere bereits beschriebene, jedoch nicht dargestellte Ausführungsbeispiele, beispielsweise im Sinne eine Steckverbindung, sind ohne Weiteres möglich. Auch können statt des Rings 59 Vorsprünge 31, 31' vorgesehen sein.

Der Haltering 59 weist bei dem dargestellten Ausführungsbeispiel noch zwei radiale Schlitze auf, von denen hier nur der Schlitz 63 dargestellt ist. In diesen greift der Vorsprung 13 ein. In den nicht dargestellten, gegenüberliegenden Schlitz greift entsprechend der nicht dargestellte Vorsprung 13' ein. Es ist auch möglich, in diesem Bereich keine Schlitze, sondern Wandabschnitte vorzusehen, wobei dann bevorzugt eine erhöhte Reibung im Bereich der Ansätze 17, 17' mit den Wandabschnitten auftritt. Selbstverständlich ist es auch möglich, dass die Wandabschnitte entsprechende Vertiefungen aufweisen, in welche die Ansätze 17, 17' vorzugsweise verrastend eingreifen.

Besonders bevorzugt weist mindestens eines der Haltemittel 11, 11' eine gewisse Elastizität auf, und die Abmessungen der Haltemittel 11, 11' sind so gewählt, dass im verbundenen beziehungsweise verrasteten Zustand eine Vorspannkraft auf die Verbindung wirkt, welche den Behälter 1 besonders sicher, fest und stabil an der Haltevorrichtung 27 hält. Es wird deutlich, dass auf ein Gehäusevorderteil zur Aufnahme des Behälters 1 verzichtet werden kann. Die Injektionshilfe 55 ist daher besonders einfach aufgebaut.

Bei bekannten Injektionshilfen, welche ein Gehäusevorderteil zur Aufnahme des Behälters 1 aufweisen, ist typischerweise an einem distalen Ende desselben ein Verbindungselement vorgesehen, um ein Injektionselement, beispielsweise eine Kanüle, mit der Injektionshilfe 55 zu verbinden.

Bei dem dargestellten Ausführungsbeispiel ist bevorzugt an dem Verschluss 49 des Behälters 1 ein Verbindungselement 65 vorgesehen. Es ist hier als Gewinde 67 ausgebildet. Dieses kann mit einem korrespondierenden, an einem Injektionselement vorgesehenen Gewinde kämmen. Der Verschluss 49 weist im Übrigen ein Dichtelement auf, welches hier als Septum 69 ausgebildet ist.

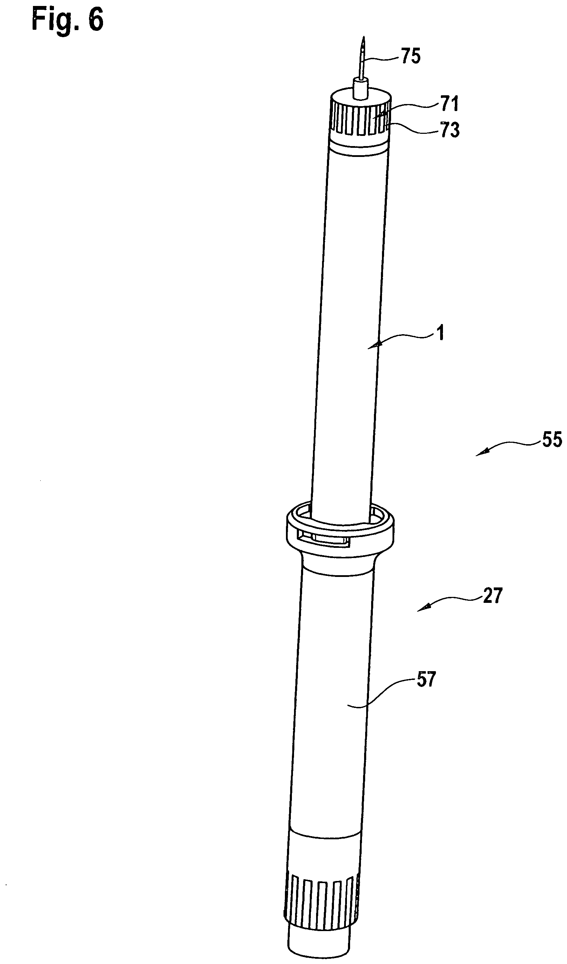

Figur 6 zeigt die Injektionshilfe 55 gemäß Figur 5 in zusammengesetztem Zustand. Gleiche und funktionsgleiche Elemente sind mit gleichen Bezugszeichen versehen, so dass insofern auf die Vorangegangene Beschreibung verwiesen wird. Im Bereich des hier nicht dargestellten Verschlusses 49 ist ein Injektionselement 71 angeordnet. Dieses weist ein nicht dargestelltes Innengewinde auf, welches mit dem ebenfalls nicht dargestellten Außengewinde 67des Verschlusses 49 kämmt. Das Injektionselement 71 umfasst ein Halteelement 73 mit dem entsprechenden Innengewinde sowie eine von dem Halteelement 73 gehaltene beziehungsweise an diesem angeordnete Injektionsnadel 75.

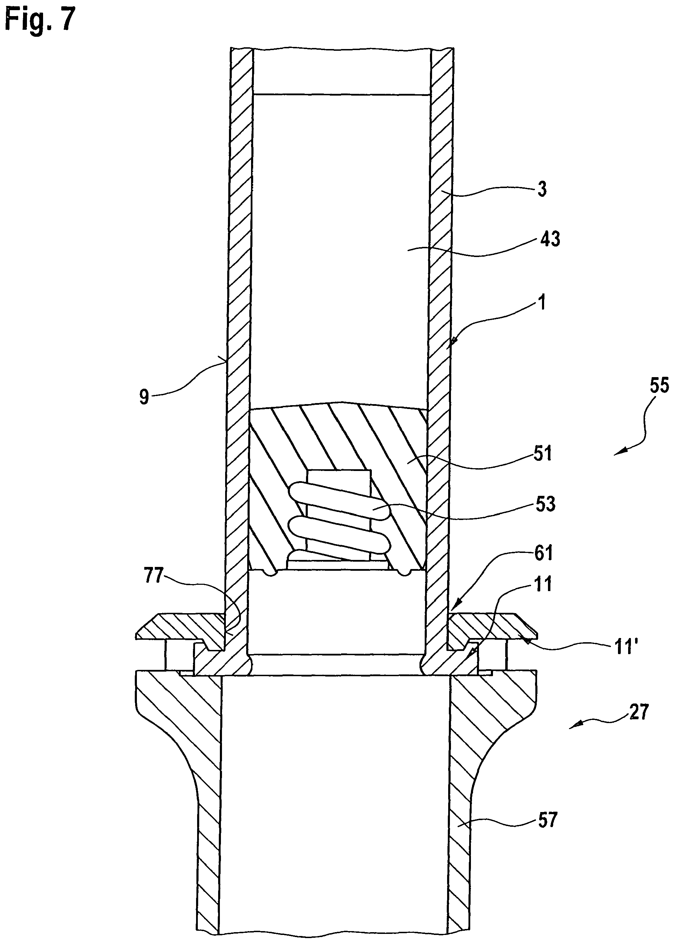

Figur 7 zeigt eine geschnittene Detailansicht der Injektionshilfe 55 gemäß Figur 5. Gleiche und funktionsgleiche Elemente sind mit gleichen Bezugszeichen versehen, so dass insofern auf die vorangegangene Beschreibung verwiesen wird. Das Mechanikteil 57 umfasst die zur Vorbereitung und Durchführung einer Injektion mit der Injektionshilfe 55 notwendigen Elemente. Ist der Behälter 1 als Doppelkammersystem ausgebildet, umfasst das Mechanikteil 57 auch die für eine Rekonstitution der in der distalen Kammer angeordneten Zubereitung notwendigen Elemente. Insbesondere umfasst das Mechanikteil 57 eine hier nicht dargestellte Kolbenstange, die vorzugsweise mit Hilfe des Gewindes 53 mit dem Endstopfen 51 in Eingriff kommt.

Um eine problemlose Vorbereitung und Durchführung der Injektion zu gewährleisten, müssen der Behälter 1 und das Mechanikteil 57 relativ zueinander zentriert sein. Dies bedeutet, dass die Längsachsen des Behälters 1 und des Mechanikteils 57 zusammenfallen müssen.

Die Zentrierung erfolgt vorzugsweise über die Öffnung 61. Wie in Figur 5 dargestellt, weist diese vorzugsweise keinen konstanten Innendurchmesser auf, sondern dieser variiert entlang des Umfangs der Öffnung 61. Gleichwohl weist sie bevorzugt einen Bereich auf, indem der Innendurchmesser in etwa dem Außendurchmesser des Grundkörpers 3 entspricht. Der Behälter 1 wird relativ zu dem Mechanikteil 57 zentriert, indem die Umfangsfläche 9 des Grundkörpers 3 an einer Innenfläche 77 der Öffnung 61 anliegt. Besonders bevorzugt ist der Innendurchmesser der Öffnung 61 bereichsweise kleiner als der Außendurchmesser des Grundkörpers 3. Entweder der Grundkörper 3 oder das Halteelement 11' ist bevorzugt im Bereich der Öffnung 61 elastisch, so dass eine mit einer Vorspannkraft beaufschlagte, reibschlüssige Verbindung im Berührungsbereich der Umfangsfläche 9 mit der Innenfläche 77 resultiert. Bevorzugt ist das Halteelement 11' in diesem Bereich elastisch ausgebildet. Insgesamt wird so eine besonders sichere Zentrierung erzielt.

Bei einem anderen Ausführungsbeispiel erfolgt die Zentrierung über die hier nicht dargestellte Kolbenstange des Mechanikteils 57. Diese weist dann vorzugsweise einen Außendurchmesser auf, welcher dem Innendurchmesser des Grundkörpers 3 zumindest im Einführungsbereich der Kolbenstange entspricht, so dass der Grundkörper 3 relativ zu dem Mechanikteil 57 zentriert wird, wenn die Kolbenstange in diesen eingeführt wird.

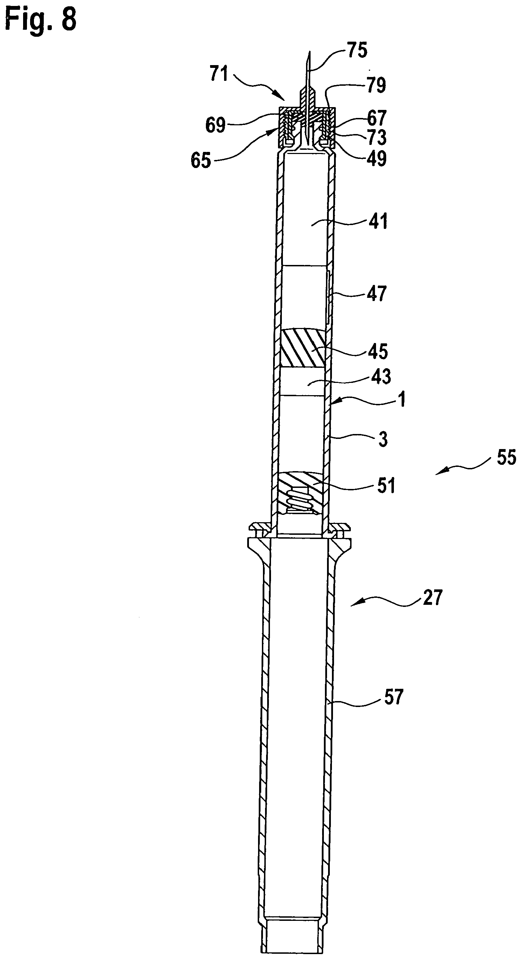

Figur 8 zeigt eine vollständige Längsschnittansicht der Injektionshilfe 55 gemäß Figur 6. Gleiche und funktionsgleiche Elemente sind mit gleichen Bezugszeichen versehen, so dass insofern auf die vorangegangene Beschreibung verwiesen wird. Hier ist insbesondere das Injektionselement 71 dargestellt. Dieses ist mit Hilfe des Verbindungselements 65 an dem Verschluss 49 angeordnet. Dabei kämmt ein Innengewinde 79 des Halteelements 73 mit dem Außengewinde 67 des Verschlusses 49.

Das Injektionselement 71 umfasst die Injektionsnadel 75, die bei dem dargestellten Ausführungsbeispiel nicht nur an ihrem distalen sondern auch an ihrem proximalen Ende spitz geschliffen ist. Sie durchsticht mit ihrem distalen Ende das Septum 69, wenn das Injektionselement 71 an dem Verschluss 49 angeordnet wird. Hierdurch wird ein Fluidpfad von der distalen Kammer 41 über die Injektionsnadel 75 in die Umgebung des Grundkörpers 3 gebildet, so dass letztlich eine in der distalen Kammer 41 angeordnete pharmazeutische Substanz injiziert werden kann.

Es zeigt sich, dass ein an dem Behälter 1 vorgesehenes Haltemittel 11 auch mit anderen als den bisher beschriebenen Ausführungsbeispielen von Haltevorrichtungen zusammenwirken kann. Beispielsweise können Anlageteile für weitere Prozessschritte, zum Beispiel das Etikettieren des Behälters 1, eine entsprechende Haltevorrichtung aufweisen, um den Behälter 1 greifen beziehungsweise halten zu können.

Auch Anbauteile zur Handhabung, Aktivierung und/oder Applikation des Behälters 1 beziehungsweise der in dem Behälter 1 angeordneten pharmazeutischen Zubereitung können als entsprechende Haltevorrichtung ausgebildet sein oder eine solche Haltevorrichtung umfassen.

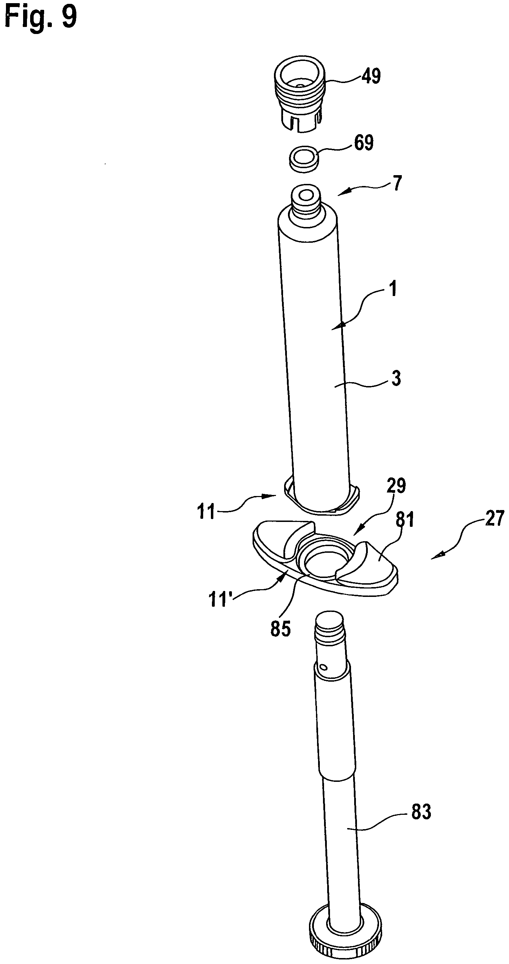

Figur 9 zeigt ein weiteres Ausführungsbeispiel einer Haltevorrichtung 27, die hier als Fingerauflage 81 ausgebildet ist. Gleiche und funktionsgleiche Elemente sind mit gleichen Bezugszeichen versehen, so dass insofern auf die vorangegangene Beschreibung verwiesen wird. In Figur 9 ist außerdem in der Explosionsdarstellung der Behälter 1 mit dem Verschluss 49, dem Dichtelement beziehungsweise Septum 69 und eine Kolbenstange 83 dargestellt. Die Fingerauflage 81 weist eine Aufnahme 29 mit einem Haltemittel 11' auf, welches mit dem Haltemittel 11 des Behälters 1 so zusammenwirken kann, dass der Grundkörper 3 des Behälters 1 sicher an der Haltevorrichtung 27 beziehungsweise der Fingerauflage 81 gehalten wird. Dabei sind die Haltemittel 11, 11' bevorzugt in einer der bereits beschriebenen Weisen ausgebildet.

Die Fingerauflage umfasst hier einen - in radialer Richtung gesehen - nach innen vorspringenden Ring 85. Dessen Innendurchmesser ist bevorzugt kleiner als der Innendurchmesser der proximalen Öffnung des Grundkörpers 3. Dadurch wirkt der Ring 85 als Rückhalteelement beispielsweise als sogenannter Backstop, welches ein Herausziehen des Endstopfens 51 aus dem Grundkörper 3 verhindert.

Ein solches Rückhalteelement für den Endstopfen 51 kann auch in anderer, an sich bekannter Weise an der Fingerauflage 81 vorgesehen sein. Es ist möglich, dass das Rückhalteelement den Endstopfen 51 nicht unmittelbar zurückhält, sondern mit der Kolbenstange 83 in einer Weise zusammenwirkt, die eine Zurückverlagerung des Endstopfens 51 über eine bestimmte Position hinaus nicht zulässt.

Der Verschluss 49 ist hier in bekannter Weise als für die Lyophilisation geeigneter Verschluss ausgebildet, der an dem Behälter 1 in einer ersten Rastposition angeordnet werden kann, in welcher ein Fluidpfad in die Umgebung des Behälters 1 freigegeben ist, um eine Lyophilisation zu ermöglichen. Er kann außerdem in einer zweiten Rastposition angeordnet werden, in welcher er dessen distales Ende 7 sicher verschließt.

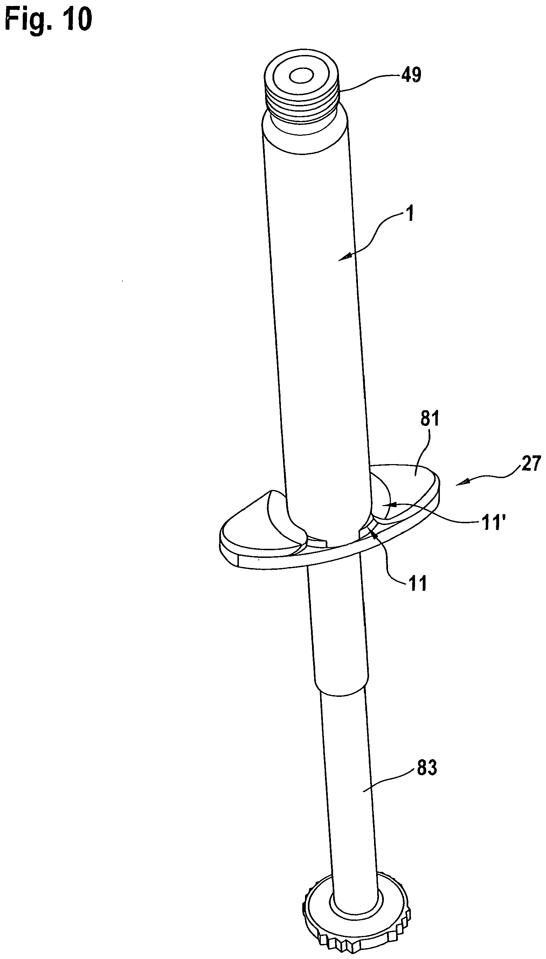

Figur 10 zeigt das Ausführungsbeispiel gemäß Figur 9 in zusammengesetztem Zustand. Gleiche und funktionsgleiche Elemente sind mit gleichen Bezugszeichen versehen, so dass insoweit auf die vorangegangene Beschreibung verwiesen wird. Die Fingerauflage 81 ist hier über die Haltemittel 11, 11' mit dem Behälter 1 verbunden. Dabei sind die Haltemittel 11, 11' vorzugsweise miteinander verrastet.

Es zeigt sich noch Folgendes: Eine Rastverbindung der Haltemittel 11, 11' kann gegebenenfalls so bewirkt werden, dass die Verbindung nicht zerstörungsfrei wieder gelöst werden kann. Dies ist insbesondere dann der Fall, wenn elastische Rastelemente zunächst beispielsweise aufgrund von Einführschrägen zurückweichen beziehungsweise zurückschwenken um anschließend in eine Hinterschneidung einzurasten. Sind die Halteelemente 11, 11' unlösbar miteinander verrastet, wird die Haltevorrichtung 27 zusammen mit dem Behälter 1 entsorgt. Sind dagegen die Halteelemente 11, 11' lösbar miteinander verbunden, kann gegebenenfalls die Haltevorrichtung 27 wiederverwendet werden, auch wenn der Behälter 1 nach seiner Verwendung entsorgt wird.

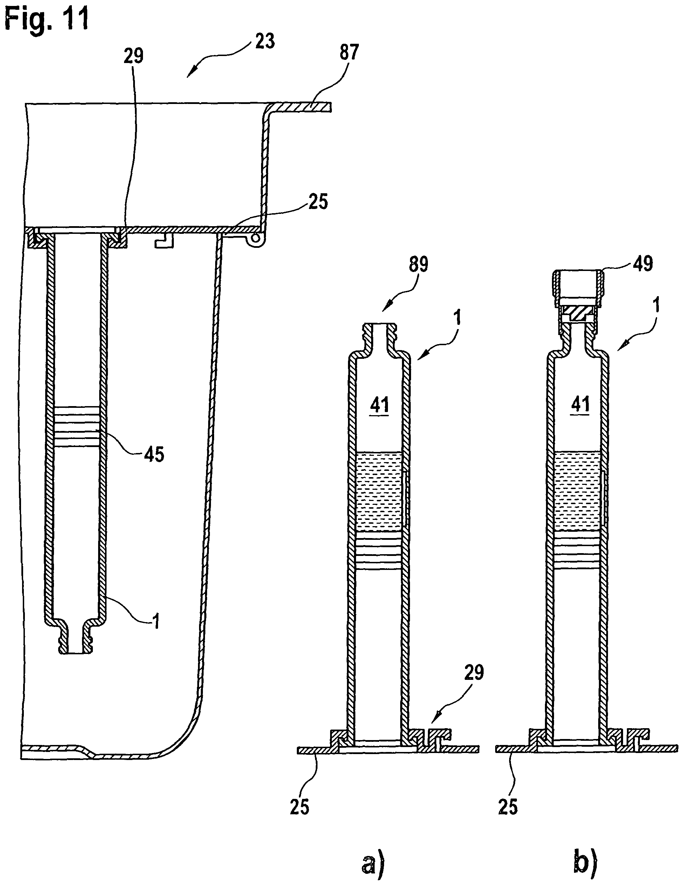

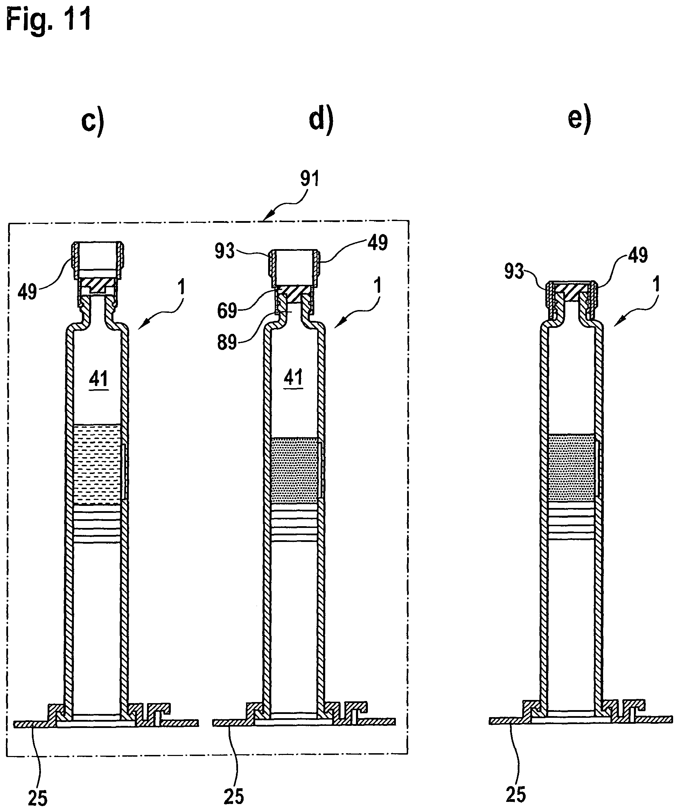

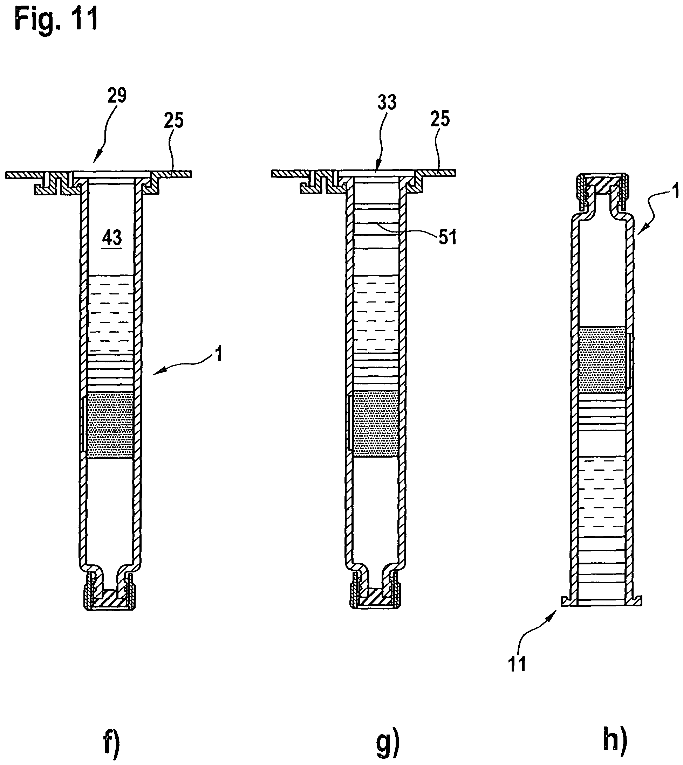

Figur 11 zeigt eine schematische Darstellung eines Verfahrens zum Befüllen eines Behälters 1, der als Doppelkammersystem ausgebildet ist, wobei eine pharmazeutische Zubereitung in der distalen Kammer 41 gefriergetrocknet wird. Gleiche und funktionsgleiche Elemente sind mit gleichen Bezugszeichen versehen, so dass insofern auf die vorangegangene Beschreibung verwiesen wird.

Ganz links in Figur 11 ist eine Haltesystem 23 dargestellt, welches ein Wannenelement 87 umfasst, in welchem das Trägersystem 25 angeordnet ist. Dieses umfasst Aufnahmen 29, in denen Behälter 1 angeordnet sind. Das Haltesystem 23 entspricht bevorzugt der Tub/Nest-Konfiguration, wobei das Wannenelement 87 als Tub und das Trägersystem 25 beziehungsweise die Haltevorrichtung 27 als Nest ausgebildet ist. Vorzugsweise ist das Wannenelement 87 mit einem nicht dargestellten Folienelement verschlossen. Nachdem das Haltesystem 23 in einen Reinraum eingebracht wurde, wird das Folienelement entfernt und damit das Wannenelement 87 geöffnet. Es ist dann möglich, durch das Trägersystem 25 hindurch den Mittelstopfen 45 in dem Behälter 1 anzuordnen. Bevorzugt wird dies - wie auch die weiteren Prozessschritte - für alle in dem Trägersystem 25 angeordneten Behälter 1 parallel durchgeführt. Die Prozessschritte werden hier allerdings beispielhaft anhand eines Behälters 1 erläutert. Das Setzen des Mittelstopfens 45 kann allerdings auch erfolgen, nachdem das Trägersystem 25 bereits aus dem Wannenelement 87 entnommen wurde.

Als nächstes wird das Trägersystem 25 gewendet, so dass die in Figur 11a) dargestellten Konfiguration resultiert. Die distale Kammer 41 wird vorzugsweise durch eine distale Öffnung 89 des Behälters 1 befüllt.

Wie in Figur 11b) dargestellt, wird dann der Verschluss 49 in an sich bekannter Weise in einer ersten Rastposition an dem Behälter 1 angeordnet, wobei ein Fluidpfad von der distalen Kammer 41 in die Umgebung des Behälters 1 bestehen bleibt.

Anschließend wird das Trägersystem 25 mit den Behältern 1 in einen hier schematisch dargestellten Gefriertrockner 91 eingebracht.

Figur 11c) zeigt den Behälter 1 vor der Gefriertrocknung. Figur 11d) zeigt den Behälter 1 nach der Gefriertrocknung, und nachdem der Verschluss 49 in seiner zweiten Rastposition angeordnet wurde, in welcher das Septum 69 beziehungsweise das entsprechende Dichtelement die Öffnung 89 dicht verschließt. Lediglich ein Sicherungsring 93 muss noch in seine Sicherungsposition gebracht werden, in der er den Verschluss 49 sicher an dem Behälter 1 hält.

Während des gesamten Prozesses, insbesondere auch während der Gefriertrocknung bleiben die Behälter 1 an dem Trägersystem 25 angeordnet. Dieses ist vergleichsweise dünn und leicht ausgebildet und umfasst vorzugsweise Kunststoff. Dies spart insbesondere beim Abkühlen und anschließenden Aufwärmen während beziehungsweise nach der Gefriertrocknung Energie, weil die Wärmekapazität des Trägersystems 25 gering ist. Im Vergleich hierzu müssen herkömmliche Behälter in massive Metall- beziehungsweise Stahlmagazine einsortiert werden, welche eine viel höhere Wärmekapazität aufweisen und im Übrigen schwerer zu handhaben sind. Ein Kunststoff umfassendes oder aus Kunststoff bestehendes Trägersystem 25 kann im Anschluss an das hier dargestellte Verfahren entsorgt werden. Die bekannten Metall- beziehungsweise Stahlmagazine müssen dagegen aufwendig gereinigt und sterilisiert beziehungsweise autoklaviert werden. Auch insoweit ermöglicht also das Trägersystem 25 eine einfachere Prozessführung. Vorzugsweise umfasst auch das Wannenelement 87 Kunststoff beziehungsweise besteht aus diesem.

In Figur 11e) ist das Trägersystem 25 mit den Behältern 1 aus dem Gefriertrockner 91 ausgeschleust. Der Sicherungsring 93 ist nun in seiner Sicherungsposition angeordnet, so dass der Verschluss 49 sicher und fest an dem Behälter 1 angeordnet ist.

Um die proximale Kammer 43 wie in Figur 11f) dargestellt zu befüllen, müssen die Behälter 1 gewendet werden. Dies geschieht hier einfach dadurch, dass das Trägersystem 25 gewendet wird. Da die Behälter 1 sicher in den Aufnahmen 29 angeordnet sind, können sie nicht herausfallen und es ist ohne weiteres möglich, die Behälter 1 gemeinsam mit Hilfe des Trägersystems 25 zu wenden.

Schließlich wird der Endstopfen 51 in dem in Figur 11g) gezeigten Schritt durch das Trägersystem 25 beziehungsweise die Öffnung 33 hindurch gesetzt.

Figur 11h) zeigt den Behälter 1, der nun nicht mehr mit dem Trägersystem 25 in Eingriff ist. Er wird bevorzugt noch einer Endkontrolle unterzogen, bevor er einer weiteren Verwendung zugeführt wird. Wie bereits beschrieben, kann dabei das Haltemittel 11 genutzt werden, um den Behälter 1 mit weiteren Haltevorrichtungen beispielsweise einer Etikettieranlage oder mit einer Injektionshilfe in Eingriff zu bringen.

Insgesamt zeigt sich, dass der vorgeschlagene Behälter 1, die vorgeschlagene Haltevorrichtung 27, das vorgeschlagene Haltesystem 23, sowie die vorgeschlagene Injektionshilfe 55 sich durch eine besonders sichere und stabile Verbindung auszeichnen, die mit Hilfe von Haltemitteln 11, 11' bewirkt wird. Dadurch wird die Handhabung auch mehrerer Behälter 1 deutlich vereinfacht. Außerdem werden kosmetische Defekte an den Behältern 1 sicher vermieden. Die Injektionshilfe 55 ist einfach und ohne großen logistischen Aufwand herstellbar und daher kostengünstig.

## Patentansprüche

1. Haltevorrichtung für Behälter für pharmazeutische Zubereitungen mit einer Mehrzahl von Aufnahmen (29,29') für Behälter (1), wobei jede der Aufnahmen (29,29') mindestens ein Haltemittel (11') umfasst, welches so ausgebildet ist, dass es mit mindestens einem korrespondierenden Haltemittel (11) eines Behälters (1) für pharmazeutische Zubereitungen so zusammenwirken kann, dass ein Grundkörper (3) des Behälters (1) sicher an der Haltevorrichtung (27) gehalten wird, wobei die Haltevorrichtung (27) als Trägersystem (25) für ein Haltesystem (23) für pharmazeutische Behälter (1) ausgebildet ist, **dadurch gekennzeichnet, dass** das Haltemittel (11') mindestens einen hakenförmigen Vorsprung (31,31') und/oder mindestens eine Ausnehmung umfasst, wobei der mindestens eine hakenförmige Vorsprung (31,31') und/oder die mindestens eine Ausnehmung einen axialen, das heißt sich entlang einer Längsrichtung eines bestimmungsgemäß in der Aufnahme angeordneten Behälters (1) erstreckenden, Ansatz (35) und eine korrespondierende axiale Vertiefung (37) aufweist, wobei das Haltemittel (11') eingerichtet ist, um mit dem korrespondierenden Haltemittel (11) nach Art eines Bajonettverschlusses zusammenzuwirken, wobei der Bajonettverschluss eine Steck-Dreh-Mechanik umfasst, bei der das korrespondierende Haltemittel (11) in einem in Axialrichtung in das Haltemittel (11`) eingesteckten Zustand um die Axialrichtung gedreht wird, wobei die Haltevorrichtung (27) mindestens eine Öffnung (33) aufweist, durch welche eine Kammer eines in der Aufnahme (29,29') angeordneten Behälters (1) zugänglich ist, und wobei die Öffnung (33) eingerichtet ist, um die Kammer zu befüllen.

2. Haltevorrichtung nach Anspruch 1, **dadurch gekennzeichnet, dass** die Haltevorrichtung (27) als ein als Nest ausgebildetes Trägersystem (25) für ein Haltesystem (23) für pharmazeutische Behälter (1) ausgebildet und eingerichtet ist zur Anordnung in einem als Tub ausgebildeten Wannenelement (87).

3. Haltevorrichtung nach einem der vorhergehenden Ansprüche, **dadurch gekennzeichnet, dass** im Bereich der Öffnung (33) zwei sich - in Umfangsrichtung gesehen - gegenüberliegende, hakenförmige Vorsprünge (31,31') vorgesehen sind.

4. Haltesystem für Behälter für pharmazeutische Zubereitungen mit einem Trägersystem (25), **dadurch gekennzeichnet, dass** das Trägersystem (25) als Haltevorrichtung (27) nach einem der Ansprüche 1 bis 3 ausgebildet ist.

5. Haltesystem nach Anspruch 4, **gekennzeichnet durch** ein Wannenelement (87), in welchem das Trägersystem (25) anordenbar ist.

## Claims

1. Holding device for containers for pharmaceutical preparations with a plurality of receptacles (29,29') for containers (1), wherein each of the receptacles (29,29') comprises at least one holding means (11') which is configured such that it can cooperate with at least one corresponding holding means (11) of a container (1) for pharmaceutical preparations such that a base body (3) of the container (1) is held securely on the holding device (27), wherein the holding device (27) is configured as a carrier system (25) for a holding system (23) for pharmaceutical containers (1), **characterised in that** the holding means (11') comprises at least one hook-shaped projection (31,31') and/or at least one recess, wherein the at least one hook-shaped projection (31, 31') and/or the at least one recess has an axial, that means extending along a longitudinal direction of a container (1) arranged in the receptacle as intended, edge (35) and a corresponding axial depression (37), wherein the holding means (11') is arranged to cooperate with the corresponding holding means (11) in the manner of a bayonet catch, wherein the bayonet catch comprises a plug-and-rotate mechanism, wherein the corresponding holding means (11) is rotated about the axial direction in a state inserted into the holding means (11') in the axial direction, wherein the holding device (27) has at least one opening (33) through which a chamber of a container (1) located in the receptacle (29, 29') is accessible, and wherein the opening (33) is arranged to fill the chamber.

2. Holding device according to claim 1, **characterised in that** the holding device (27) is configured as a nest-shaped carrier system (25) for a holding system (23) for pharmaceutical containers (1) and is adapted for arrangement in a tub-shaped tub element (87).

3. Holding device according to one of the preceding claims, **characterised in that**, in the area of the opening (33), two opposing - when seen in the circumferential direction - hook-shaped projections (31,31') are provided.

4. Holding system for containers for pharmaceutical preparations with a carrier system (25), **characterised in that** the carrier system (25) is configured as a holding device (27) according to one of claims 1 to 3.

5. Holding system according to claim 4, **characterized by** a tub element (87) in which the carrier system (25) can be arranged.

## Revendications

1. Dispositif de maintien pour récipients de préparations pharmaceutiques comprenant une pluralité de logements (29, 29') pour récipients (1), chacun des logements (29, 29') comprenant au moins un moyen de maintien (11') qui est réalisé de manière à pouvoir coopérer avec au moins un moyen de maintien (11) correspondant d'un récipient (1) de préparations pharmaceutiques de telle sorte, qu'un corps de base (3) du récipient (1) est maintenu de manière sûre sur le dispositif de maintien (27), le dispositif de maintien (27) étant conçu comme un système de support (25) pour un système de maintien (23) pour des récipients pharmaceutiques (1), **caractérisé en ce que** le moyen de maintien (11') comprend au moins une saillie en forme de crochet (31, 31') et/ou au moins un évidement, la au moins une saillie en forme de crochet (31, 31') et/ou le au moins un évidement présentant un appendice axial (35), c'est-à-dire s'étendant le long d'une direction longitudinale d'un récipient (1) disposé dans le logement conformément à sa destination, et un renfoncement axial correspondant (37), le moyen de maintien (11') étant conçu pour coopérer avec le moyen de maintien correspondant (11) à la manière d'une fermeture à baïonnette, dans lequel la fermeture à baïonnette comprend un mécanisme d'enfichage et de rotation par lequel le moyen de maintien correspondant (11) est tourné autour de la direction axiale dans un état enfiché dans le moyen de maintien (11') dans la direction axiale, dans lequel le dispositif de maintien (27) présente au moins une ouverture (33) à travers laquelle une chambre d'un récipient (1) disposé dans le logement (29, 29') est accessible, et dans lequel l'ouverture (33) est conçue pour remplir la chambre.

2. Dispositif de maintien selon la revendication 1, **caractérisé en ce que** le dispositif de maintien (27) est réalisé comme un système de support (25) réalisé sous forme de nid pour un système de maintien (23) pour des récipients pharmaceutiques (1) et est conçu pour être disposé dans un élément de cuve (87) réalisé sous forme de tube.

3. Dispositif de maintien selon l'une des revendications précédentes, **caractérisé en ce que** deux saillies (31, 31') en forme de crochets, opposées l'une à l'autre - vue dans la direction périphérique - sont prévues dans la zone de l'ouverture (33).

4. Système de maintien pour récipients de préparations pharmaceutiques avec un système de support (25), **caractérisé en ce que** le système de support (25) est réalisé comme un dispositif de maintien (27) selon l'une des revendications 1 à 3.

5. Système de maintien selon la revendication 4, **caractérisé par** un élément de cuve (87) dans lequel le système de support (25) peut être disposé.
